# EUROPEAN PATENT APPLICATION

(11) **EP 2 280 274 A2**
(43) Date of publication of application: **02.02.2011**
(21) Application number: 10185830.6
(22) Date of filing: 17.12.2004
(51) Int. Cl.: G01N 29/02, G01N 33/543, B01L 3/00

(54) **Acousto-mechanical detection system**

(30) Priority: 30.12.2003 US 533169 P
(62) Divisional of application: 04821309.4
(71) Applicant: 3M Innovative Properties Co., St. Paul, MN 55133-3427 (US)
(72) Inventor: Carter, Chad J., St. Paul, MN 55133-3427 (US); David, Moses M., St. Paul, MN 55133-3427 (US); Dodge, Larry H., St. Paul, MN 55133-3427 (US); Free, Benton M., St. Paul, MN 55133-3427 (US); Gason, Samuel J., St. Paul, MN 55133-3427 (US); Huizinga, John S., St. Paul, MN 55133-3427 (US); Johnston, Raymond P., St. Paul, MN 55133-3427 (US); Lakshmi, Brinda B., St. Paul, MN 55133-3427 (US); Mach, Patrick A., St. Paul, MN 55133-3427 (US); Martin, Larry G., St. Paul, MN 55133-3427 (US); Pekurovsky, Mikhail L., St. Paul, MN 55133-3427 (US); Schaberg, Mark S., St. Paul, MN 55133-3427 (US); Smith, Jeffrey D., St. Paul, MN 55133-3427 (US); Wood, Kenneth B., St. Paul, MN 55133-3427 (US); Xu, Wenyuan, St. Paul, MN 55133-3427 (US)
(74) Representative: Vossius & Partner

(57) **Abstract**

Detection systems and methods for detecting target biological analytes within sample material using acousto-mechanical energy generated by a sensor are disclosed. The acousto-mechanical energy may be provided using an acousto-mechanical sensor, *e.g*., a surface acoustic wave sensor such as, *e.g*., a shear horizontal surface acoustic wave sensor (*e.g*., a LSH-SAW sensor), A variety of techniques for modifying the effective mass of the target biological analytes in sample material are disclosed, including fractionating or disassembling the target biological analytes in the sample material (*e.g*., lysing the target biological analyte), adding a detectable mass to the target biological analyte or enhancing coupling of the target biological analyte (*e.g*., through the use of magnetic particles), exposing the sample material to a reagent that causes a change in at least detectable physical property in the sample material if the target biological analyte is present (*e.g*., a change in viscous, elastic, and/or viscoelastic properties), etc.

## Description

### RELATED APPLICATIONS

The present application claims priority to U.S. Provisional Patent Application Serial No. 60/533,169, filed on December 30,2003, which is hereby incorporated by reference in its entirety.

### GOVERNMENT RIGHTS

The U.S. Government may have certain rights to this invention under the terms of DAAD 13-03-C-0047 granted by Department of Defense.

The present invention relates to systems and methods for detecting one or more target biological analytes using acousto-mechanical energy.

Unlike classical clinical assays such as tube and slide coagulase tests, the detection cartridges of the present invention employ an integrated sensor. As used herein "sensor" refers to a device that detects a change in at least one physical property and produces a signal in response to the detectable change. The manner in which the sensor detects a change may include, e.g., electrochemical changes, optical changes, electro-optical changes, acousto-mechanical changes, etc. For example, electrochemical sensors utilize potentiometric and amperometric measurements, whereas optical sensors may utilize absorbance, fluorescence, luminescence and evanescent waves.

In the case of acousto-mechanical sensors, many biological analytes are introduced to the sensors in combination with a liquid carrier. The liquid carrier may undesirably reduce the sensitivity of the acousto-mechanical detection systems. Furthermore, the selectivity of such sensors may rely on properties that cannot be quickly detected, e.g., the test sample may need to be incubated or otherwise developed over time.

To address that problem, selectivity can be obtained by binding a target biological analyte to, e.g., a detection surface. Selective binding of known target biological analytes to detection surfaces can, however, raise issues when the sensor used relies on acousto-mechanical energy to detect the target biological analyte.

One technical problem that is not addressed is the affect of the size and relative low level of mechanical rigidity of many or most biological analytes. This issue may be especially problematic in connection with shear-horizontal surface acoustic wave detectors.

Shear horizontal surface acoustic wave sensors are designed to propagate a wave of acousto-mechanical energy along the plane of the sensor detection surface. In some systems, a waveguide may be provided at the detection surface to localize the acousto-mechanical wave at the surface and increases the sensitivity of the sensor (as compared to a non-waveguided sensor). This modified shear horizontal surface acoustic wave is often referred to as a Love-wave shear horizontal surface acoustic wave sensor ("LSH-SAW sensor").

Such sensors have been used in connection with the detection of chemicals and other materials where the size of the target analytes is relatively small. As a result, the mass of the target analytes is largely located within the effective wave field of the sensors (e.g., about 60 nanometers (nm) for a sensor operating at, e.g., a frequency of 103 Megahertz (MHZ) in water).

What has not heretofore been appreciated is that the effective wave field of the sensors is significantly limited relative to the size of biological analytes to be detected. For example, biological analytes that are found, e.g., in the form of single cell microorganisms, may have a typical diameter of, e.g., about 1 micrometer (1000 nm). As noted above, however, the effective wave field of the sensors is only about 60 nm. As a result, significant portions of the mass of the target analyte may be located outside of the effective wave field of the sensors.

In addition to the size differential, the target biological analytes are often membranes filled with various components including water. As a result, the effect of acousto-mechanical energy traveling within the effective wave field above a sensor on the total mass of the biological analytes can be significantly limited. In many instances, target biological analytes attached to the surfaces of such sensors cannot be accurately distinguished from the liquid medium used to deliver the target analytes to the detector.

Although not wishing to be bound by theory, it is theorized that the relative lack of mechanical rigidity in biological analytes attached to a detection surface, i.e., their fluid nature, may significantly limit the amount of mass of the biological analytes that is effectively coupled to the detection surface. In other words, although the biological analytes may be attached to the detection surface, a significant portion of the mass of the biological analyte is not acoustically or mechanically coupled to the acousto-mechanical wave produced by the sensor. As a result, the ability of an acousto-mechanical sensor (e.g., a LSH-SAW sensor) to effectively detect the presence or absence of target biological analytes can be severely limited.

### SUMMARY OF THE INVENTION

The present invention provides detection systems, methods for detecting target biological analytes within sample material using acousto-mechanical energy generated by a sensor, and components that may be used in such systems and methods. The acousto-mechanical energy may preferably be provided using an acousto-mechanical sensor, e.g., a surface acoustic wave sensor such as, e.g., a shear horizontal surface acoustic wave sensor (e.g., a LSH-SAW sensor), although other acousto-mechanical sensor technologies may be used in connection with the systems and methods of the present invention in some instances.

As discussed above, one issue that may be raised in the use of acousto-mechanical energy to detect the presence or absence of target biological analyte in sample material is the ability to effectively couple the mass of the target biological analyte to the detection surface such that the acousto-mechanical energy from the sensor is affected in a detectable manner. The detection systems and methods of the present invention may, in some embodiments, provide a variety of techniques for modifying the effective mass of the target biological analytes in sample material. The mass-modification techniques may include, e.g., fractionating or disassembling the target biological analytes in the sample material (e.g., lysing the target biological analyte), adding a detectable mass to the target biological analyte or enhancing coupling of the target biological analyte (e.g., through the use of magnetic particles), exposing the sample material to a reagent that causes a change in at least detectable physical property in the sample material if the target biological analyte is present (e.g., a change in viscous, elastic, and/or viscoelastic properties), etc.

Use of effective mass-modification techniques may, in some embodiments of the present invention, provide acousto-mechanical biosensors that may produce rapid, accurate results in the detection of various target biological analytes. As used herein, "target biological analyte" may include, e.g., microorganisms (e.g., bacteria, viruses, endospores, fungi, protozoans, etc.), proteins, peptides, amino acids, fatty acids, nucleic acids, carbohydrates, hormones, steroids, lipids, vitamins, etc.

The target biological analyte may be obtained from sample material that is or that includes a test specimen obtained by any suitable method and may largely be dependent on the type of target biological agent to be detected. For example, the test specimen may be obtained from a subject (human, animal, etc.) or other source by e.g., collecting a biological tissue and/or fluid sample (e.g., blood, urine, feces, saliva, semen, bile, ocular lens fluid, synovial fluid, cerebral spinal fluid, pus, sweat, exudate, mucous, lactation milk, skin, hair, nails, etc.). In other instances, the test specimen may be obtained as an environmental sample, product sample, food sample, etc. The test specimen as obtained may be a liquid, gas, solid or combination thereof.

Before delivery to the systems and methods of the present invention, the sample material and/or test specimen may be subjected to prior treatment, e.g., dilution of viscous fluids, concentration, filtration, distillation, dialysis, addition of reagents, chemical treatment, etc.

Potential advantages of the systems and methods of the present invention are the presentation of sample materials (which may include, e.g., test specimens, reagents, carrier fluids, buffers, etc.) to the detection surface of a sensor in a controlled manner that may enhance detection and/or reproducibility.

The controlled presentation may include control over the delivery of sample material to the detection surface. The control may preferably be provided using a module-based input system in which sample materials such as, e.g., test specimens, reagents, buffers, wash materials, etc. can be introduced into the detection cartridge at selected times, at selected rates, in selected orders, etc.

Controlled presentation may also include control over the fluid flow front progression across the detection surface. The "flow front", as that term is used herein, refers to the leading edge of a bolus of fluid moving across a detection surface within a detection chamber. A potential advantage of control over the flow front progression is that preferably all of the detection surface maybe wetted out by the sample material, i.e., bubbles or voids in the fluid that could occupy a portion of the detection surface may preferably be reduced or eliminated.

Controlled presentation may also encompass volumetric flow control through a detection chamber that, in some embodiments of the present invention, maybe achieved by drawing fluid through the detection chamber using, e.g., capillary forces, porous membranes, absorbent media, etc. Controlling the flow rate of sample material over the detection surface may provide advantages. If, for example, the flow rate is too fast, target analyte in the sample material may not be accurately detected because selective attachment may be reduced or prevented. Conversely, if the flow rate is too slow, excessive non-specific binding of non-targeted analytes or other materials to the detection surface may occur, thereby potentially providing a false positive signal.

The systems and methods of the present invention may also include sealed modules that may be selectively incorporated into, e.g., a detection cartridge, to facilitate the detection of different target analytes within the detection cartridge. Before use, the modules may preferably be sealed to prevent materials located therein from escaping and/or to prevent contamination of the interior volume of the modules. The modules may preferably include two or more isolated chambers in which different constituents may be stored before they are introduced to each other and to the detection cartridges. The introduction and mixing of the different constituents, along with their introduction into the detection cartridge (and, ultimately, the sensor) may be controlled using the modules and actuators. Isolated storage of many different constituents may greatly enhance the shelf-life of materials that may be used to assist in the detection of target analytes. Some reagents that may benefit from isolated dry storage conditions may include, e.g., lysing reagents, fibrinogen, assay-tagged particles (e.g., magnetic particles), etc.

The modules may be selected and attached to the detection cartridge by the manufacturer or, in some instances, by an end user. The flexibility offered to an end user to, essentially, customize a detection cartridge at the point-of-use may offer additional advantages in terms of economy and efficiency. For example, different modules containing different reagents, buffers, etc. may be supplied to the end-user for their selective combination of modules in a detection cartridge to perform a specific assay for a specific target analyte.

Although the exemplary embodiments described herein may include a single sensor, the detection cartridges of the present invention may include two or more sensors, with the two or more sensors being substantially similar to each other or different. Furthermore, each sensor in a detection cartridge according to the present invention may include two or more channels (e.g., a detection channel and a reference channel). Alternatively, different sensors may be used to provide a detection channel and a reference channel within a detection cartridge. If multiple sensors are provided, they may be located in the same detection chamber or in different detection chambers within a detection cartridge.

In some embodiments, the acousto-mechanical sensors may include enhanced pathlengths. Potential advantages of pathlength-enhanced acousto-mechanical sensors may include, e.g., increased magnitude and phase sensitivity to viscous, elastic, and viscoelastic changes in the presence of sample material and/or target analyte.

The present invention may be utilized in combination with various materials, methods, systems, apparatus, etc. as described in various U.S. patent applications identified below, all of which are incorporated by reference in their respective entireties. They include U.S. Patent Application Serial Nos. 60/533,162, filed on December 30, 2003; 60/533,178, filed on December 30, 2003; 10/896,392, filed July 22, 2004; 10/713,174, filed November 14, 2003; 10/987,522, filed November 12, 2004; 10/714,053, filed November 14,2003; 10/987,075, filed November 12, 2004; 60/533,171, filed December 30, 2003; 10/960,491, filed October 7, 2004; 60/533,177, filed December 30,2003; 60/533,176, filed December 30, 2003; _, titled "Method of Enhancing Signal Detection of Cell-Wall Components of Cells", filed on even date herewith (Attorney Docket No. 59467US002); _,titled "Soluble Polymers as Amine Capture Agents and Methods", filed on even date herewith (Attorney Docket No. 59995US002); _, titled "Multifunctional Amine Capture Agents", filed on even date herewith (Attorney Docket No. 59996US002); PCT Application No. _, titled "Estimating Propagation Velocity Through A Surface Acoustic Wave Sensor", filed on even date herewith (Attorney Docket No. 58927WO003); PCT Application No. titled "Surface Acoustic Wave Sensor Assemblies", filed on even date herewith (Attorney Docket No. 58928WO003); PCT Application No. _, titled "Detection Cartridges, Modules, Systems and Methods", filed on even date herewith (Attorney Docket No. 60342WO003); and PCT Application No. _, titled "Acoustic Sensors and Methods", filed on even date herewith (Attorney Docket No. 60209WO003).

In one aspect, the present invention provides a system for detecting a target biological analyte. The system includes a surface acoustic wave sensor with a detection surface; a capture agent located on the detection surface, wherein the capture agent is capable of selectively attaching the target biological analyte to the detection surface; a detection chamber located within an interior volume of a housing, the detection chamber including a volume defined by the detection surface and an opposing surface spaced apart from and facing the detection surface, wherein the opposing surface of the detection chamber includes a flow front control feature; a waste chamber located within the interior volume of the housing, the waste chamber in fluid communication with the detection chamber; means for driving the shear horizontal surface acoustic wave sensor; means for analyzing data from the surface acoustic wave sensor to determine if target biological analyte is coupled to the capture agent.

In another aspect, the present invention provides a system for detecting a target biological analyte, the system including a shear horizontal surface acoustic wave sensor comprising a detection surface; a capture agent located on the detection surface, wherein the capture agent is capable of selectively attaching the target biological analyte to the detection surface; a detection chamber located within an interior volume of a housing, the detection chamber having a volume defined by the detection surface and an opposing surface spaced from and facing the detection surface, wherein the opposing surface of the detection chamber includes a flow control feature; a waste chamber in fluid communication with the detection chamber, wherein absorbent material is located within the waste chamber; capillary structure located between the detection chamber and the waste chamber; at least one module having an exit port attached to the housing through a module port that opens into the interior volume of the housing, wherein the at least one module contains a selected reagent within a chamber, and further wherein the at least one module includes an exit seal closing the exit port of the at least one module, a plunger located within the at least one module, wherein the plunger is movable from a loaded position in which the plunger is distal from the exit port to an unloaded position in which the plunger is proximate the exit port, wherein movement of the plunger towards the exit port opens the exit seal and delivers material from the chamber of the at least one module into the interior volume of the housing through the exit port; an actuator operably coupled to the plunger of the at least one module, wherein the actuator is capable of moving the plunger from the loaded position to the unloaded position; means for driving the shear horizontal surface acoustic wave sensor; and means for analyzing data from the shear horizontal surface acoustic wave sensor to determine if the target biological analyte is coupled to the capture agent.

In another aspect, the present invention provides a method of detecting a target biological analyte using a system of the invention, the method including contacting sample material with a mass-modification agent, wherein a target biological analyte within the sample material interacts with the mass-modification agent such that a mass-modified target biological analyte is obtained within the test sample; contacting the detection surface of the surface acoustic wave device with the mass-modified test sample by delivering the test sample to the detection chamber; selectively attaching the mass-modified target biological analyte to the detection surface; and operating the surface acoustic wave device to detect the attached mass-modified biological analyte while the detection surface is submersed in liquid.

In another aspect, the present invention provides a method of detecting a biological analyte, the method including fractionating target biological analyte located within sample material; contacting a detection surface of a shear horizontal surface acoustic wave sensor with the sample material containing the fractionated target biological analyte; selectively attaching the fractionated target biological analyte to the detection surface; and operating the shear horizontal surface acoustic wave sensor to detect the attached fractionated target biological analyte while the detection surface is submersed in liquid.

In another aspect, the present invention provides a shear horizontal surface acoustic wave sensor including a piezoelectric substrate with a major surface; at least one transducer on the major surface of the piezoelectric substrate, wherein the at least one transducer defines an acoustic path on the major surface of the piezoelectric substrate, wherein the acoustic path has a first end and a second end; wherein the at least one transducer has a contact pad on the major surface of the piezoelectric substrate, wherein the contact pad is located off to a first side of the acoustic path and between the first end and the second end of the acoustic path, wherein the contact pad is connected to the at least one transducer by a lead.

These and other features and advantages of the detection systems and methods of the present invention may be described in connection with various illustrative embodiments of the invention below.

### BRIEF DESCRIPTION OF THE DRAWINGS

FIG. 1 is a schematic diagram of one exemplary detection system according to the present invention.
FIG. 2 is a schematic diagram of another exemplary detection system according to the present invention.
FIG. 3 is a schematic diagram of one exemplary detection cartridge according to the present invention.
FIG. 4A is a plan view of one exemplary opposing surface including flow front control features according to the present invention.
FIG. 4B is a perspective view of another exemplary opposing surface including flow front control features according to the present invention.
FIG. 4C is a cross-sectional view of another exemplary opposing surface including flow front control features according to the present invention.
FIG. 4D is a cross-sectional view of another exemplary opposing surface including flow front control features according to the present invention.
FIG. 4E is a cross-sectional view of another exemplary opposing surface including flow front control features according to the present invention.
FIG. 4F is a plan view of another exemplary opposing surface including flow front control features according to the present invention.
FIG. 5 is a plan view of an opposing surface including flow control features in the form of hydrophobic and hydrophilic regions.
FIG. 6 is a plan view of another exemplary opposing surface including flow control features according to the present invention.
FIG. 7 is a plan view of another exemplary opposing surface including flow control features according to the present invention.
FIG. 8 is a schematic diagram of one exemplary detection cartridge according to the present invention.
FIG. 8A is an enlarged cross-sectional view of an alternative exemplary opening into a waste chamber in a detection cartridge according to the present invention.
FIG. 8B is an exploded diagram of the components depicted in FIG. 8A.
FIG. 8C is an enlarged plan view of an acoustic sensor including two channels on a detection surface, wherein the channels have an enhanced acoustic pathlength.
FIG. 9A depicts one alternative connection between a detection chamber and a waste chamber in a detection cartridge according to the present invention,
FIG. 9B is a cross-sectional view of the flow passage of FIG. 9A taken along line 9B-9B.
FIG. 10A is a cross-sectional diagram of one exemplary module that may be used in connection with the present invention.
FIG. 10B is a cross-sectional diagram of the module of FIG. 10A during use.
FIG. 10C is an enlarged partial cross-sectional view of an alternative plunger and tip seated in the unloaded position within a module of the present invention, FIG. 10D is a cross-sectional view taken along line 10D-10D in FIG. 10C.
FIG. 11 is a schematic diagram of one system that may be used in connection with the present invention.
FIG. 12 is a schematic diagram of the construction of one exemplary acousto-mechanical sensor that may be used in connection with the present invention.

### DETAILED DESCRIPTION OF EXEMPLARY EMBODIMENTS OF THE INVENTION

In the following detailed description of exemplary embodiments of the invention, reference is made to the accompanying figures of the drawing which form a part hereof, and in which are shown, by way of illustration, specific embodiments in which the invention may be practiced. It is to be understood that other embodiments may be utilized and structural changes may be made without departing from the scope of the present invention.

### EFFECTIVE MASS-MODIFICATION

As discussed herein, effective detection of target biological analyte in sample material using acousto-mechanical biosensors may rely on modification of the effective detectable mass of the target biological analyte within the sample material. Some mass-modification techniques used in connection with the present invention may include, but are not limited to, e.g., fractionating or disassembling the target biological analyte in the sample material, adding a detectable mass to the target biological analyte, exposing the sample material to a reagent that causes a change in at least detectable physical property in the test sample if the target biological analyte is present.

### Fractionating/Disassembling:

The mass modification of the target biological analyte in connection with the systems and methods of the present invention may preferably take the form of, e.g., fractionating or otherwise disassembling the target biological analyte into smaller fragments or particles such that an increased percentage of the mass of the target biological analyte can be retained within the effective wave field of the acousto-mechanical sensor and/or effectively coupled with the detection surface of the acousto-mechanical sensor.

The fractionating or disassembly may be accomplished chemically, mechanically, electrically, thermally, or through combinations of two or more such techniques. Examples of some potentially suitable chemical fractionating techniques may involve, e.g., the use of one or more enzymes, hypertonic solutions, hypotonic solutions, detergents, etc. Examples of some potentially suitable mechanical fractionating techniques may include, e.g., exposure to sonic energy, mechanical agitation (e.g., in the presence of beads or other particles to enhance breakdown), etc. Thermal fractionating may be performed by, e.g., heating the target biological agent. Other fractionating/disassembly techniques may also be used in connection with the present invention.

U.S. Patent Application No. _, titled "Method of Enhancing Signal Detection of Cell-Wall Components of Cells", and filed on even date herewith (Attorney Docket No. 59467US002) describes the use of lysing as one method of fractionating a target biological analyte that may be used in connection with the present invention.

### Particle Attachment:

In another approach, mass-modification of the target biological analyte in connection with the systems and methods of the present invention may take the form of adding detectable mass to a target biological analyte using, e.g., magnetic particles, etc. with selective affinity to the target biological analyte. A wide variety of particles may be attached to the target biological analyte, e.g., inorganic particles, organic particles, etc. Some potentially suitable particles may include, e.g., silica, titania, alumina, latex, etc. The particles may be attached in combination with fractionating/disassembly techniques (where, e.g., the particles could attach to fragments of a cell wall, etc.). In other instances, the particles may be used alone, i.e., in the absence of intentional fractionating/disassembly of the target biological analyte. The particles may selectively attach to the target biological analyte or they may non-selectively attach to materials within a test sample.

It may be preferred, however, that particles attached to the target biological analyte (or fragments thereof) may be magnetic such that they can be acted on by a magnetic field. In such a system, a magnetic field may be established proximate the detection surface such that the mass-modified target biological analytes are attracted and attached to the detection surface where they can be detected by the acousto-mechanical sensor.

Magnetic particles can enhance detection of the target biological analyte in a number of ways. The magnetic particles may be used to drive the attached target biological analyte to the detection surface under the influence of a magnetic field, thus potentially speeding up capture and/or increasing capture efficiency. The attached magnetic particles themselves may also provide additional mass to the target biological analyte to enhance detection, as well as potentially adding additional magnetic force to the weight force exerted by the target biological analyte itself if the magnetic field is active during the detection process. In other instances, the magnetic particles may modify the mechanical rigidity of the target biological analyte, thereby potentially rendering the target biological analyte more easily detectable by the acousto-mechanical sensor.

General methods of using magnetic particles and methods of making magnetic particles may be described in, e.g., U.S. Patent No. 3,970,518 (Giaever); U.S. Patent No. 4,001,197 (Mitchell et al.); and EP Publication No. 0016552 (Widder et al.). These methods may be adapted for use in connection with the present invention.

### Sample Material Property Change:

In yet another approach, the mass-modification may involve exposing the sample material to a reagent that causes a change in at least detectable physical property in the sample material if the target biological analyte is present. The detectable physical change maybe characterized as mass-modification because it may preferably increase the effective detectable mass of the target biological analyte. Such a change may be, e.g., a change in the viscous, elastic, and/or viscoelastic properties of the sample material in contact with the detection surface. Although a change in such properties may not technically be considered a change in mass, they can change the effective detectable mass of the sample material because the mass located within the effective wave field can be more easily detected if one or more such properties are changed.

Examples of some suitable mass-modification techniques may be, e.g., the use of fibrinogen in combination with staphylococcus as described in, e.g., U.S. Patent Application Serial No. 60/533,171, filed on December 30, 2003 and U.S. Patent Application Serial No. 10/960,491, filed on October 7, 2004.

### ACOUSTO-MECHANICAL SENSORS

The systems and methods of the present invention preferably detect the presence of target biological analyte in a test sample through the use of acousto-mechanical energy that is measured or otherwise sensed to determine wave attenuation, phase changes, frequency changes, and/or resonant frequency changes.

The acousto-mechanical energy may be generated using, e.g., piezoelectric-based surface acoustic wave (SAW) devices. As described in, e.g., U.S. Patent No. 5,814,525 (Renschler et al.), the class of piezoelectric-based acoustic mechanical devices can be further subdivided into surface acoustic wave (SAW), acoustic plate mode (APM), or quartz crystal microbalance (QCM) devices depending on their mode of detection.

In some embodiments, the systems and methods of the present invention may be used to detect a target biological analyte attached to a detection surface. In other embodiments, the systems may be used to detect a physical change in a liquid (e.g., an aqueous solution), such as, e.g., changes in the viscous, elastic and/or viscoelastic properties that may be indicative of the presence of the target biological analyte. The propagation velocity of the surface wave is a sensitive probe that may be capable of detecting changes such as mass, elasticity, viscoelasticity, conductivity and dielectric constant in a medium in contact with the detection surface of an acousto-mechanical sensor. Thus, changes in one or more of these (or potentially other) physical properties may result in changes in the attenuation of the surface acoustic wave.

APM devices operate on a similar principle to SAW devices, except that the acoustic wave used can be operated with the device in contact with a liquid. Similarly, an alternating voltage applied to the two opposite electrodes on a QCM (typically AT-cut quartz) device induces a thickness shear wave mode whose resonance frequency changes in proportion to mass changes in a coating material.

The direction of the acoustic wave propagation (e.g., in a plane parallel to the waveguide or perpendicular to the plane of the waveguide) may be determined by the crystal-cut of the piezoelectric material from which the biosensor is constructed. SAW biosensors in which the majority of the acoustic wave propagates in and out of the plane (e.g., Rayleigh wave, most Lamb-waves) are typically not employed in liquid sensing applications because of acoustic damping from the liquid in contact with the surface.

For liquid sample mediums, a shear horizontal surface acoustic wave biosensor (SH-SAW) may preferably be used. SH-SAW sensors are typically constructed from a piezoelectric material with a crystal-cut and orientation that allows the wave propagation to be rotated to a shear horizontal mode, i.e., parallel to the plane defined by the waveguide, resulting in reduced acoustic damping loss to a liquid in contact with the detection surface. Shear horizontal acoustic waves may include, e.g., thickness shear modes (TSM), acoustic plate modes (APM), surface skimming bulk waves (SSBW), Love-waves, leaky acoustic waves (LSAW), and Bleustein-Gulyaev (BG) waves.

In particular, Love wave sensors may include a substrate supporting a SH wave mode such as SSBW of ST quartz or the leaky wave of 36°YXLiTaO₃. These modes may preferably be converted into a Love-wave mode by application of thin acoustic guiding layer or waveguide. These waves are frequency dependentand can be generated if the shear wave velocity of the waveguide layer is lower than that of the piezoelectric substrate.

Waveguide materials may preferably be materials that exhibit one or more of the following properties: low acoustic losses, low electrical conductivity, robustness and stability in water and aqueous solutions, relatively low acoustic velocities, hydrophobicity, higher molecular weights, highly cross-linked, etc. In one example, SiO₂ has been used as an acoustic waveguide layer on a quartz substrate. Examples of other thermoplastic and crosslinked polymeric waveguide materials include, e.g., epoxy, polymethylmethacrylate, phenolic resin (e.g., NOVALAC), polyimide, polystyrene, etc.

Alternatively, QCM devices can also be used with liquid sample mediums. Biosensors employing acousto-mechanical devices and components may be described in, e.g., U.S. Patent Nos. 5,076,094 (Frye et al.); 5,117,146 (Martin et al.); 5,235,235 (Martin et al.); 5,151,110 (Bein et al.); 5,763,283 (Cernosek et al.); 5,814,525 (Renschler et al.); 5,836,203 ((Martin et al.); and 6,232,139 (Casalnuovo et al.). Shear horizontal SAW devices can be obtained from various manufacturers such as Sandia Corporation, Albuquerque, New Mexico. Some SH-SAW biosensors that may be used in connection with the present invention may also described in Branch et al., "Low-level detection of a Bacillus anthracis simulant using Love-wave biosensors on 36°YX LiTaO3," Biosensors and Bioelectronics (accepted 22 August 2003).

The various documents identified herein may all describe potentially suitable means for driving the sensors of the present invention and means for analyzing data from the sensors to determine whether a target material is attached to the sensor surface.

### SELECTIVE ATTACHMENT

The detection systems and methods of the present invention may preferably provide for the selective attachment of target biological analyte to the detection surface or to another component that can be coupled to the detection surface. Regardless of whether the selective attachment of the target biological analyte is to the detection surface itself or to another component, it may be preferred that the mass coupled to the detection surface be capable of detection using acousto-mechanical energy.

Selective attachment may be achieved by a variety of techniques. Some examples may include, e.g., antigen-antibody binding; affinity binding (e.g., avidin-biotin, nickel chelates, glutathione-GST); covalent attachment (e.g., azlactone, trichlorotriazine, phosphonitrilic chloride trimer or N-sulfonylaminocarbonyl-amide chemistries); etc.

The selective attachment of a target biological analyte may be achieved directly, i.e., the target biological analyte may itself be selectively attached to the detection surface. Examples of some such direct selective attachment techniques may include the use of capture agents, e.g., antigen-antibody binding (where the target biological analyte itself includes the antigen bound to an antibody immobilized on the detection surface), DNA capture, etc.

Alternatively, the selective attachment may alternatively be indirect, i.e., where the target biological analyte is selectively attached to a carrier that is selectively attached or attracted to the detection surface. One example of an indirect selective attachment technique may include, e.g., selectively binding magnetic particles to the target biological analyte such that the target biological analyte can be magnetically attracted to and retained on the detection surface.

In connection with selective attachment, it may be preferred that systems and methods of the present invention provide for low non-specific binding of other biological analytes or materials to, e.g., the detection surface. Non-specific binding can adversely affect the accuracy of results obtained using the detection systems and methods of the present invention. Non-specific binding can be addressed in many different manners. For example, biological analytes and materials that are known to potentially raise non-specific binding issues may be removed from the test sample before it is introduced to the detection surface. In another approach, blocking techniques may be used to reduce non-specific binding on the detection surface.

Because selective attachment may often rely on coatings, layers, etc. located on the acousto-mechanical detection surface, care must be taken that these materials are relatively thin and do not dampen the acousto-mechanical energy to such a degree that effective detection is prevented.

Another issue associated with selective attachment is the use of what are commonly referred to as "immobilization" technologies that may be used to hold or immobilize a capture agent on the surface of, e.g., the acousto-mechanical sensor itself. The detection systems and methods of the present invention may involve the use of a variety of immobilization technologies.

As discussed herein, the general approach is to coat or otherwise provide the detection surface of an acousto-mechanical sensor device with capture agents such as, e.g., antibodies, peptides, aptamers, or any other capture agent that has high affinity for the target biological analyte. The surface coverage and packing of the capture agent on the surface may determine the sensitivity of the sensor. The immobilization chemistry that links the capture agent to the detection surface of the sensor may play a role in the packing of the capture agents, preserving the activity of the capture agent (if it is a biomolecule), and may also contribute to the reproducibility and shelf-life of the sensor.

If the capture agents are proteins or antibodies, they can nonspecifically adsorb to a surface and lose their ability (activity) to capture the target biological analyte. A variety of immobilization methods may be used in connection with acousto-mechanical sensors to achieve the goals of high yield, activity, shelf-life and stability. These capture agents may preferably be coated using glutaraldehyde crosslinking chemistries, entrapment in acrylamide, or general coupling chemistries like carbodiimide, epoxies, cyano bromides etc.

Apart from the chemistry that binds to the capture agent and still keeps it active, there are other surface characteristics of any immobilization chemistries used in connection with the present invention that may need to be considered and that may become relevant in an acousto-mechanical sensor application. For example, the immobilization chemistries may preferably cause limited damping of the acousto-mechanical energy such that the immobilization chemistry does not prevent the sensor from yielding usable data. The immobilization chemistry may also determine how the antibody or protein is bound to the surface and, hence, the orientation of the active site of capture. The immobilization chemistry may preferably provide reproducible characteristics to obtain reproducible data and sensitivity from the acousto-mechanical sensors of the present invention.

Some immobilization technologies that may be used in connection with the systems and methods of the present invention may be described in, e.g., U.S. Patent Application Serial Nos. 10/713,174, filed November 14, 2003; 10/987,522, filed on November 12, 2004; 60/533,162, filed on December 30,2003; 60/533,178, filed on December 30, 2003,10/896,392, filed on July 22, 2004; 10/714,053, filed on November 14, 2003; 10/987,075, filed on November 12,2004; _, titled "Soluble Polymers as Amine Capture Agents and Methods", filed on even date herewith (Attorney Docket No. 59995US002); _, titled "Multifunctional Amine Capture Agents", filed on even date herewith (Attorney Docket No. 59996US002); and PCT Application No. , titled "Acoustic Sensors and Methods, filed on even date herewith (Attorney Docket No. 60209WO003).

Immobilization approaches may include a tie layer between the waveguide on an acousto-mechanical substrate and the immobilization layer. One exemplary tie layer may be, e.g., a layer of diamond-like glass, such as described in International Publication No. WO 01/66820 A1 (David et al.). Diamond-like glass is typically considered an amorphous material that includes carbon, silicon, and one or more elements selected from hydrogen, oxygen, fluorine, sulfur, titanium, or copper. Some diamond-like glass materials are formed from a tetramethylene silane precursor using a plasma process. A hydrophobic material can be produced that is further treated in an oxygen plasma to control the silanol concentration on the surface. Other tie layers such as, e.g., gold, may also be used.

Diamond-like glass can be in the form of a thin film or in the form of a coating on another layer or material in the substrate. In some applications, the diamond-like glass can be in the form of a thin film having at least 30 weight percent carbon, at least 25 weight percent silicon, and up to 45 weight percent oxygen. Such films can be flexible and transparent. In some multilayer substrates, the diamond-like glass is deposited on a layer of diamond-like carbon. The diamond-like carbon can, in some embodiments, function as a second tie layer or primer layer between a polymeric layer and a layer of diamond-like glass in a multilayer substrate. Diamond-like carbon films can be prepared, for example, from acetylene in a plasma reactor. Other methods of preparing such films are described U.S. Patent Nos. 5,888,594 and 5,948,166 (both to David et al.), as well as in the article by M. David et al., A1ChE Journal, 37 (3), 367-376 (March 1991).

### EXEMPLARY DETECTION SYSTEMS/METHODS

Some illustrative exemplary embodiments of systems and methods according to the principles of the present invention are described below in connection with various figures.

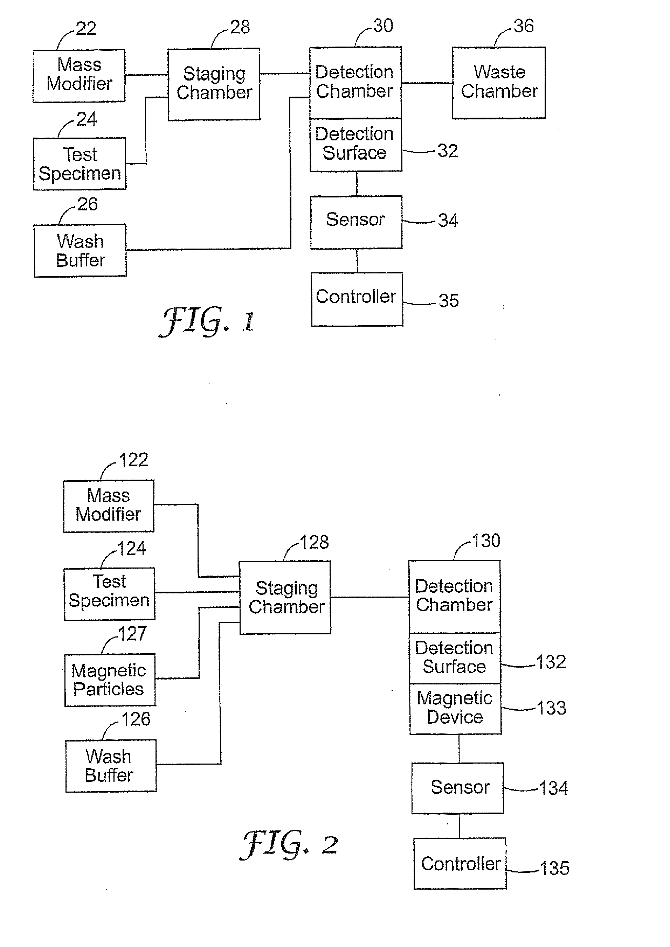

FIG. 1 is a schematic diagram of one detection system 10 according to the present invention that may include inputs in the form of a mass-modifying agent 22, test specimen 24, and wash buffer 26. These various components may be introduced into, e.g., a staging chamber 28 where the various components may intermix and/or react with each other to form sample material that can be further processed. For example, it may be desirable that the mass-modifying agent 22 and test specimen 24 be introduced into the staging chamber 28 to allow the agent 22 to act on the test specimen 24 such that any target biological analyte within the test specimen 24 can be effectively modified. Alternatively, one or more these components may be present in the preparation chamber 28 before one or more of the other components are introduced therein.

It may be preferred that the mass-modifying agent 22 be selective to the target biological analyte, i.e., that other biological analytes in the test specimen 24 are not modified by the agent 22. Alternatively, the mass-modifying agent 22 may be non-selective, i.e., it may act on a number of biological analytes in the test specimen 24, regardless of whether the biological analytes are the target biological analyte or not.

In some embodiments, the mass-modifying agent 22 may preferably be a chemical fractionating agent such as, e.g., one or more enzymes, hypertonic solutions, hypotonic solutions, detergents, etc. In place of fractionating, the agent 22 may be add mass through the use of particle attachment to the target biological analyte or the mass-modifying agent ma be used to cause a detectable change in a physical property based on the presence (or absence) of one or more target biological analytes in the test specimen. For example, the agent 22 may be, e.g., fibrinogen in a system/method such as that discussed in, e.g., U.S. Patent Application Serial No. 60/533,171, filed December 30, 2003 and U.S. Patent Application Serial No. 10/960,491, filed on October 7, 2004.

After mass-modification of the target biological analyte in the test specimen 24, the agent 22 and test specimen 24 may be moved from the staging chamber 28 to the detection chamber 30 where the target biological analyte can contact the detection surface 32. In the depicted system, the detection surface 32 may preferably be of the type that includes capture agents located thereon such that the target biological analyte in the sample material is selectively attached to the detection surface 32.

In various systems and methods of the present invention, e.g., that depicted in FIG. 1, it may be beneficial to provide some control over sample introduction to, flow rate over, and dwell time on the detection surface 32. In some instances, for example, it may be desirable to prevent the introduction of gas bubbles to the detection surface 32 when the sample material is in liquid form. Another sample material control issue may be, e.g., controlling the flow rate of the sample material over the detection surface 32. If the flow rate is too fast, the target biological analyte in the sample material may not be accurately detected because selective attachment may be reduced or prevented. Conversely, if the flow rate is too slow, excessive non-specific binding of non-targeted biological analytes or other materials to the detection surface 32 may occur.

Fluid control on the detection surface may be addressed by a variety of techniques (either alone or in combination). Potential approaches include, e.g., surface flow control (using channels or other features), material properties (e.g., using hydrophilic or hydrophobic materials, coatings, etc.), using porous membranes to control flow towards or away from the detection surface, etc.

After the target biological analytes in the sample material have been resident in the detection chamber 30 for a sufficient period of time or have moved therethrough, a wash buffer 26 may be introduced into the detection chamber 30 to remove unattached biological analytes and other materials from the detection chamber 30. These materials may preferably move into a waste chamber 36 connected to the detection chamber 30.

Detection of any target biological analytes selectively attached to the detection surface preferably occurs using the sensor 34 as operated by a control module 35. The control module 35 may preferably operate the sensor 34 such that the appropriate acousto-mechanical energy is generated and also monitor the sensor 34 such that a determination of the presence or absence of the target biological analyte on the detection surface 32 can be made.

Examples of techniques and means for driving and monitoring acousto-mechanical sensors (as delay lines devices, resonators, etc.) such as those that may be used in connection with the present invention may be found in, e.g., U.S. Patent Nos, 5,076,094 (Frye et al.); 5,117,146 (Martin et al.); 5,235,235 (Martin et al.); 5,151,110 (Bein et al.); 5,763,283 (Cernosek et al.); 5,814,525 (Renschler et al.); 5,836,203 ((Martin et al.); and 6,232,139 (Casalnuovo et al.), etc. Further examples may be described in, e.g., Branch et al., "Low-level detection of a Bacillus anthracis simulant using Love-wave biosensors on 36°YX LiTaO3," Biosensors and Bioelectronics (accepted 22 August 2003); as well as in U.S. Patent Application Serial No. 60/533,177, filed on December 30, 2003 and PCT Application No. _, titled "Estimating Propagation Velocity Through A Surface Acoustic Wave Sensor", filed on even date herewith (Attorney Docket No. 58927WO003).

An alternative exemplary detection system 110 is depicted in FIG. 2 and includes inputs in the form of a mass-modification agent 122, test specimen 124, wash buffer 126, and magnetic particles 127. These various components may be introduced into, e.g., a staging chamber 128 where the various components may intermix and/or react with each other. Alternatively, one or more these components may be present in the staging chamber 128 before one or more of the other components are introduced therein.

For example, it may be desirable that a mass-modification agent 122 and the test specimen 124 be introduced into the staging chamber 128 to allow the agent 122 to act on and/or attach to the target biological analyte within the test specimen 124. Following that, the magnetic particles 127 may be introduced into the staging chamber 128. The magnetic particles 127 may preferably selectively attach to the target biological analyte material within the staging chamber 128 although selective attachment may not be necessary.

In some instances, the use of magnetic particles 127 may themselves serve as a mass-modifying agent by adding mass to the attached target biological analyte as discussed above. In such a system, the magnetic particles 127 may reduce or eliminate the need for a separate mass modification agent 122 in the system of FIG. 2 if the magnetic particles 127 alone are sufficient to improve the response of the sensor.

The attachment of biological analytes to, e.g., magnetic particles, may be described generally in, e.g., International Publication Nos. WO 02/090565 (Ritterband) and WO 00/70040 (Bitner et al.) which describe the use of magnetic beads in kits to concentrate cells, as well as magnetically responsive particles. Selective attachment of a biological agent to magnetic particles (e.g., paramagnetic microspheres) is also described in, e.g., Kim et al., "Impedance characterization of a piezoelectric immunosensor part II: Salmonella typhimurium detection using magnetic enhancement," Biosensors and Bioelectronics 18 (2003) 91-99.

After attachment of the target biological analyte in the test specimen 124 to the magnetic particles 127, the sample material (with the test specimen 124 and associated magnetic particles) may be moved from the staging chamber 128 to the detection chamber 130 where the target biological analyte in the sample material can contact the detection surface 132. Because the target biological analyte is associated with magnetic particles, it may be desirable to include a magnetic field generator 133 capable of generating a magnetic field at the detection surface 132 such that the target biological analyte associated with magnetic particles can be retained on the detection surface for detection using sensor 134 operated by controller 135. In other words, the magnetic forces provided by the magnetic field proximate the detection surface 132 may draw the magnetic particles (and attached target biological analyte) to the detection surface 132. The magnetic field generator 133 may be any suitable device that can provide a magnetic field arranged to draw magnetic particles to the detection surface, e,g., a permanent magnet, electromagnet, etc.

The use of magnetic particles in connection with the target biological analyte may enhance detection by, e.g., moving the target biological analyte to the detection surface 132 more efficiently and/or rapidly than might be expected in the absence of, e.g., magnetic attractive forces. In addition, if the magnetic field is maintained during the actual detection process (when acoustic energy is being generated and detected), the magnetic forces may also enhance detection of the target biological analyte.

If the detection surface 132 includes selective capture agents located thereon such that the target biological analyte is selectively attached to the detection surface 132 in the absence of magnetic fields, then the magnetic particles that are not carrying (or being carried by) any target biological analyte can be removed from the detection surface 132 by, e.g., removing the magnetic field and washing the detection surface 132. Washing the detection surface 132 in the absence of a magnetic field may preferably remove magnetic particles that are not carrying (or being carried by) target biological analytes. Further, the target biological analyte (and the magnetic particles that are associated therewith) may preferably be retained on the detection surface 132 after washing in the absence of a magnetic field by the selective capture agent or agents on the detection surface 132.

Other methods of removing non-associated magnetic particles, i.e., magnetic particles that are not associated with any target biological analyte, may be performed before introducing the associated magnetic particles (i.e., magnetic particles carrying or being carried by target biological analyte).

### DETECTION CARTRIDGES

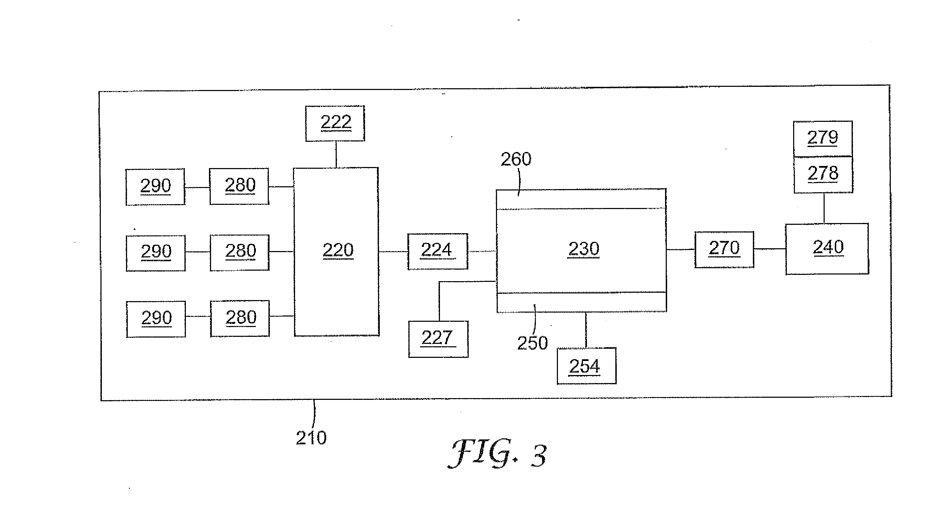

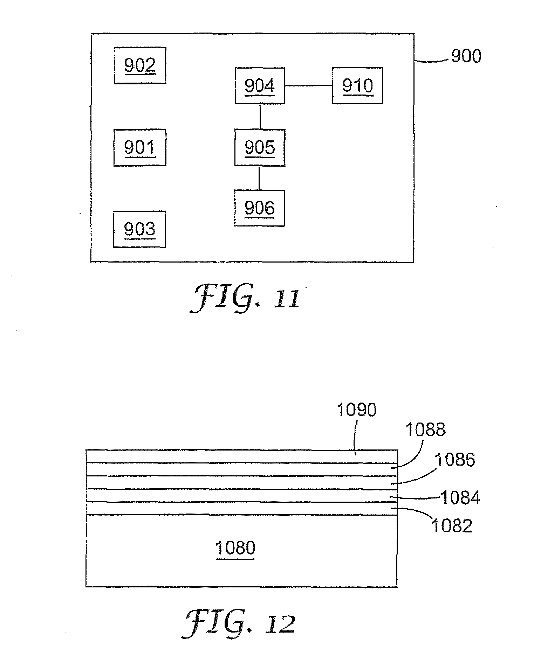

Although two exemplary systems that may be used in connection with the present invention are discussed above, various components that may be well-suited to use in such systems will now be described in more detail. Those components include, e.g., an exemplary detection cartridge depicted schematically in FIG. 3. One example of a sealed module that may be used in connection with, e.g., the detection cartridges, is depicted in connection with FIGS. 11 A & 11B. The sealed module may be used to store and/or introduce various components such as fractionating/disassembly agents, magnetic particles, reagents, wash buffers, etc. into systems of the present invention. PCT Application No. _, titled "Detection Cartridges, Modules, Systems and Methods", filed on even date herewith (Attorney Docket No. 60342WO003) may describe additional features of detection cartridges and/or modules that may be used in connection with the present invention.

In one aspect, the systems and methods of the present invention may use detection cartridges that include an integrated sensor and fluid control features that assist in selective delivery of a sample analyte to the sensor. The exemplary detection cartridge 210 depicted schematically in FIG. 3 includes, among other things, a staging chamber 220, detection chamber 230, waste chamber 240, sensor 250, volumetric flow control feature 270, and modules 280. In general, the detection cartridge 210 of FIG. 3 may be described as having an interior volume that includes the staging chamber 220, detection chamber 230 and waste chamber 240, with the different chambers defining a downstream flow direction from the staging chamber 220 through the detection chamber 230 and into the waste chamber 240. As a result, the detection chamber 230 may be described as being upstream from the waste chamber 240 and the staging chamber 220 may be described as being upstream from the detection chamber 230. Not every detection cartridge used in connection with the present invention may necessarily include the combination of components contained in detection cartridge 210 of FIG. 3.

The detection chamber 230 of the detection cartridge 210 preferably defines an interior volume between the detection surface of the sensor 250 and an opposing surface 260 located opposite from the detection surface of the sensor 250. The detection chamber 230 may preferably provide sidewalls or other structures that define the remainder of the interior volume of the detection chamber 230 (i.e., that portion of the detection chamber 230 that is not defined by the detection surface of the sensor 250 and the opposing surface 260).

Also depicted in FIG. 1 is a connector 254 that may preferably be operably connected to the sensor 250 to supply, e.g., power to the sensor 250. The connector 254 may preferably supply electrical energy to the sensor 250, although in some embodiments the connector may be used to supply optical energy or any other form of energy required to operate the sensor 250. The connector 254 may also function to connect the sensor 250 to a controller or other system that may supply control signals to the sensor 250 or that may receive signals from the sensor 250. If necessary, the connector 254 (or additional connectors) may be operably connected to other components such as valves, fluid monitors, temperature control elements (to provide heating and/or cooling), temperature sensors, and other devices that may be included as a part of the detection cartridge 210.

In addition to the detection chamber 230, the detection cartridge 210 depicted in FIG. 3 also includes an optional waste chamber 240 into which material flows after leaving the detection chamber 230. The waste chamber 240 may be in fluid communication with the detection chamber 230 through a volumetric flow control feature 270 that can be used to control the rate at which sample material from the detection chamber 230 flows into the waste chamber 240. The volumetric flow control feature 270 may preferably provide a pressure drop sufficient to draw fluid through the detection chamber 230 and move it into the waste chamber 240. In various exemplary embodiments as described herein, the volumetric flow control feature 270 may include one or more of the following components: one or more capillary channels, a porous membrane, absorbent material, a vacuum source, etc. These different components may, in various embodiments, limit or increase the flow rate depending on how and where they are deployed within the cartridge 210. For example, a capillary structure may be provided between the detection chamber 230 and the waste chamber 240 to limit flow from the detection chamber 230 into the waste chamber 240 if, e.g., the waste chamber 240 includes absorbent material that might cause excessively high flow rates in the absence of a capillary structure.

Another feature depicted in FIG. 3 is a vent 278 that may preferably be provided to place the interior volume of the detection cartridge 210 in fluid communication with the ambient atmosphere (i.e., the atmosphere in which the detection cartridge 210 is located) when the vent 278 is an open condition. The vent 278 may also preferably have a closed condition in which fluid flow through the vent 278 is substantially eliminated. Closure of the vent 278 may, in some embodiments, effectively halt or stop fluid flow through the interior volume of the detection cartridge 210. Although depicted as leading into the waste chamber 240, one or more vents may be provided and they may be directly connected to any suitable location within the detection cartridge 210, e.g., staging chamber 220, detection chamber 230, etc. The vent 278 may take any suitable form, e.g., one or more voids, tubes, fittings, etc.

The vent 278 may include a closure element 279 in the form of include a seal, cap, valve, or other structure(s) to open, close or adjust the size of the vent opening. In some embodiments, the closure element 279 may be used to either open or close the vent. In other embodiments, the closure element 279 may be adjustable such that the size of the vent opening may be adjusted to at least one size between fully closed and fully open to adjust fluid flow rate through the detection cartridge 210. For example, increasing the size of the vent opening may increase fluid flow rate while restricting the size of the vent opening may cause a controllable reduction the fluid flow rate through the interior volume of the detection cartridge 210, e.g., through the staging chamber 220, detection chamber 230, etc. If the vent 278 includes multiple orifices, one or more of the orifices can be opened or closed, etc.

Although the volumetric flow rate of fluid moving through the detection chamber 230 may be controlled by the volumetric flow control feature 270, it may be preferred to provide for control over the flow front progression through the detection chamber 230. Flow front progression control may assist in ensuring that all portions of a detection surface of the sensor 250 exposed within the detection chamber 230 are covered or wetted out by the fluid of the sample material such that bubbles or voids are not formed. It may be preferred for example that the flow front progress through the detection chamber 230 in the form of a generally straight line that is oriented perpendicular to the direction of flow through the detection chamber 230.

In the exemplary embodiment depicted in FIG. 3, the flow front control features may preferably be provided in or on the opposing surface 260. This may be particularly true if the sensor 250 relies on physical properties that may be affected by the shape and/or composition of the detection surface, e.g., if the detection surface is part of a sensor that relies on acoustic energy transmission through a waveguide that forms the detection surface or that lies underneath the detection surface. Discontinuities in physical structures or different materials arranged over the detection surface may, e.g., cause the acoustic energy to propagate over the detection surface in a manner that is not conducive to accurate detection of a target analyte within the detection chamber 30. Other sensor technologies, e.g., optical, etc., may also be better-implemented using detection surfaces that do not, themselves, include physical structures or combinations of different materials to control fluid flow front progression within a detection chamber.

In view of the concerns described above, it may be preferred to provide flow front control features in or on the opposing surface 260 of the detection chamber 230 to assist in the control of fluid flow progression over the detection surface of sensor 250. Flow front control may preferably provide control over the progression of sample material over the detection surface while also reducing or preventing bubble formation (or retention) on the detection surface.

The flow front control features provided on the opposing surface 260 may preferably be passive, i.e., they do not require any external input or energy to operate while the fluid is moving through the detection chamber 230. The flow front control features may also preferably operate over a wide range of sample volumes that may pass through the detection chamber 230 (e.g., small sample volumes in the range of 10 microliters or less up to larger sample volumes of 5 milliliters or more).

It may be preferred that the opposing surface 260 and the detection surface of the sensor 250 be spaced apart from each other such that the opposing surface 260 (and any features located thereon) does not contact the detection surface of the sensor 250. With respect to acoustic sensors, even close proximity of the opposing surface 260 to the detection surface of the sensor may adversely affect the properties of the sensor operation. It may be preferred, for example, that spacing between the detection surface of the sensor 250 and the lowermost feature of the opposing surface 260 be 20 micrometers or more, or even more preferably 50 micrometers or more. For effective flow front control, it may be preferred that the distance between the lowermost feature of the opposing surface 260 and the detection surface of the sensor 250 be 10 millimeters, alternatively 1 millimeter or less, in some instances 500 micrometer or less, and in other instances 250 micrometers or less.

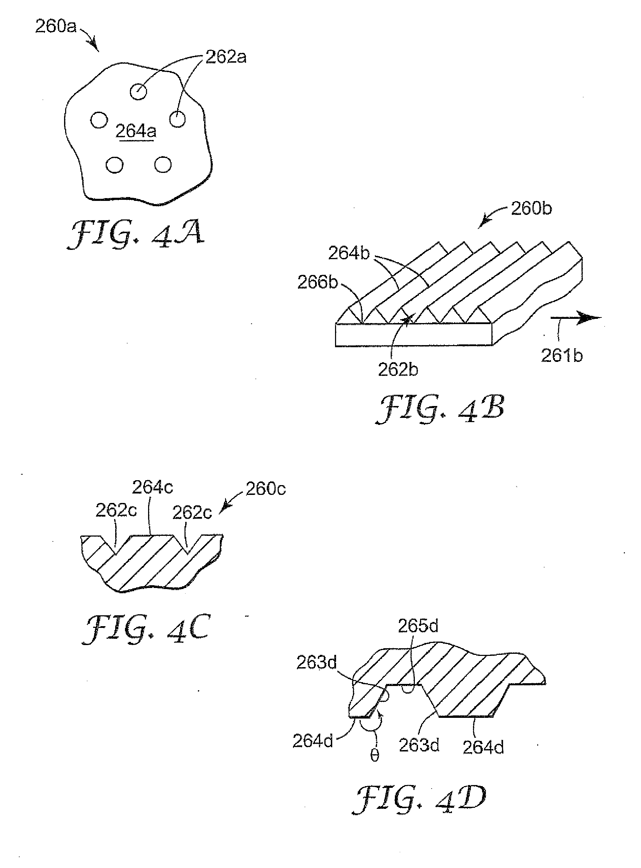

In one class of flow front control features, the opposing surface 260 may include physical structure such as channels, posts, etc. that may be used to control the flow of fluid through the detection chamber 230. Regardless of the particular physical structure, it is preferably of a large enough scale such that flow front progression through the detection chamber is meaningfully affected. FIGS. 4A-4F depict a variety of physical structures that may be used to control the flow front progression of fluid.

FIG. 4A is a plan view of one type of physical structure on an opposing surface 260a that may provide flow front control. The physical structure includes multiple discrete structures 262a, e.g., posts, embedded or attached beads, etc., dispersed over the opposing surface 260a and protruding from the land area 264a that separates the discrete structures 262a. The discrete structures 262a may be provided in any shape, e.g., circular cylinders, rectangular prisms, triangular prisms, hemispheres, etc. The height, size, spacing, and/or arrangement of the different structures 262a may be selected to provide the desired flow front control depending on fluid viscosity and/or distance between the opposing surface 260a and the detection surface within a detection chamber. It may be preferred that the structures 262a be manufactured of the same material as the land area 264a of the opposing surface 260a between the structures 262a or, alternatively, the structures 262a may be manufactured of one or more materials that differ from the materials that form the land area 264a between structures 262a.

FIG. 4B depicts another exemplary embodiment of physical structure that may be provided in connection with an opposing surface 260b. The physical structure is in the form of triangular channels 262b formed in the opposing surface 260b, with each channel 262b including two peaks 264b on either side of a valley 266b. Although the depicted channels 262b are parallel to each other and extend in a straight line that is perpendicular to the desired fluid flow (see arrow 261b in FIG. 4B), it will be understood that variations in any of these characteristics may be used if they assist in obtaining the desired flow across the detection surface. The channels 262b may be irregularly sized, irregularly shaped, irregularly spaced, straight, curved, oriented at other than a ninety degree angle to fluid flow, etc. For example, adjacent channels 262b may be immediately adjacent each other as seen in FIG. 4B. Also, although the channels 262b have a triangular cross-sectional shape, channels used in connection with the present invention may have any cross-sectional shape, e.g., arcuate, rectangular, trapezoidal, hemispherical, etc. and combinations thereof.

In other embodiments, the channels may be separated by land areas between peaks or include valleys that have a land area (i.e., that does not reach a bottom and then immediately turn upward to the adjacent peak). The land areas may be flat or take other shapes as desired. One such variation is depicted in FIG. 4C in which channels 262c in opposing surface 260c are provided with land areas 264c separating the channels 262c on opposing surface 260c.

FIG. 4D depicts another variation in physical structures that may be used for flow front control on an opposing surface 260d. The physical structures are provided in the form of channels 262d. The channels 262d of opposing surface 260d have a different shape, i.e., are more rectangular or trapezoidal, including walls 263d and roof 265d, as opposed to the triangular channels of FIGS. 4B and 4C.

Even though the channels 262d are more rectangular in shape, it may be preferred that the wall 263d at the leading edge of each channel 262d forms an angle θ (theta) with the surface 264d leading up to the channel 262d that is less than 270 degrees. As used herein, the "leading edge" of a channel is that edge that is encountered first by liquids moving in the downstream direction over the detection surface. Limiting the angle θ (theta) may promote fluid flow into the channels 262d because higher angles between the walls 263d at the leading edges and the surfaces 264d may impede fluid flow front progression. By virtue of their triangular shape, the channels in the opposing surfaces in FIGS. 4B & 4C inherently possess angles that are conducive to fluid flow into the channels.

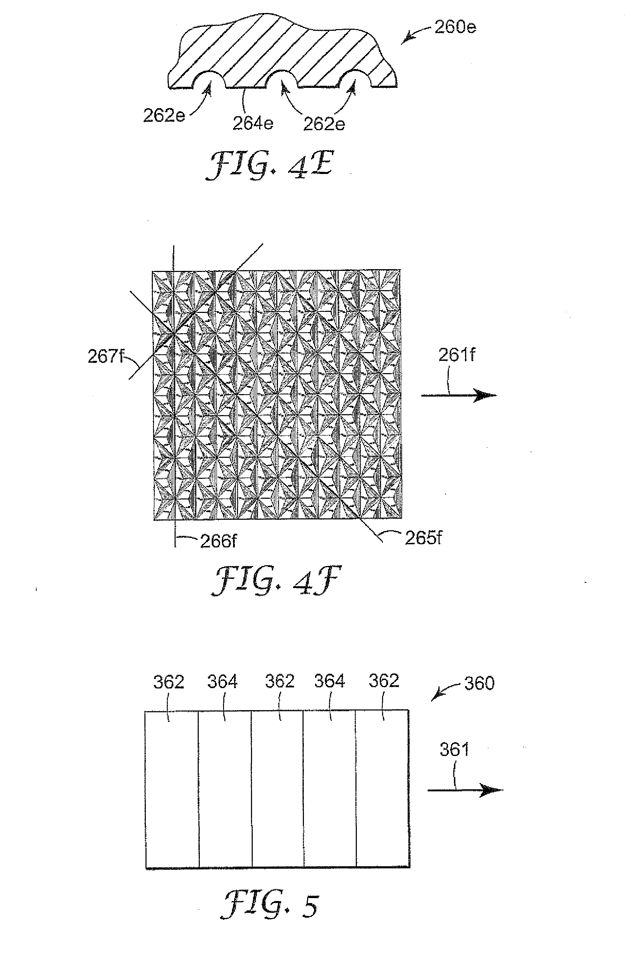

FIG. 4E depicts another embodiment of an opposing surface 260e that includes channels 262e with an arcuate (e.g., hemispherical) profile that also provide entrance angles of less than 270 degrees to also preferably promote fluid flow into the channels 262e. The channels 262e may preferably be separated by land areas 264e as depicted in FIG. 4E.

In addition to the variations described above with respect to FIGS. 4A-4E, another variation may be that channels of two or more different shapes may be provided on a single opposing surface, e.g., a mix of triangular, rectangular, hemispherical, etc. channels may be provided on the same opposing surface.

FIG. 4F depicts yet another variation of an opposing surface 260f that includes physical structure to control a fluid flow front within a detection chamber. The depicted surface 260f includes a discrete structure in the form of triangular pyramids made by a series of triangular-shaped channels formed in the surface 260f along and/or parallel to axes 265f, 266f and 267f. It may be preferred that at least one of the sets of channels be formed in a direction that is generally perpendicular to fluid flow direction as represented by arrow 261. f as, for example, the channels along and/or parallel to axis 266f Together with the angled channels along axes 265f and 267f, perpendicular channels along/parallel to axis 266f may preferably form faces on each of the pyramidal structures. Although the depicted pyramid structures have triangular bases, pyramid-shaped structures could be provided with four or more faces if so desired.

Referring again to FIG. 3, flow front control through the detection chamber 230 may also be accomplished without the use of physical structures. In some embodiments, flow front control may be accomplished through the use of hydrophilic and/or hydrophobic materials located on the opposing surface 260. FIG. 5 is a plan view of an opposing surface 360 that includes regions 362 of hydrophobic materials and regions 364 of hydrophilic materials occupying portions of the opposing surface 360. The regions 362 and 364 may preferably be provided as successive bands oriented generally perpendicular to the direction of flow through the detection chamber as illustrated by arrow 361, i.e., from an input end to an output end of a detection chamber (although other hydrophilic/hydrophobic patterns may be used). The hydrophilic and/or hydrophobic materials used in regions 362 and/or 364 may be coated or otherwise provided on the opposing surface 360. in some instances, the material used to construct the opposing surface 360 may itselfbe considered hydrophilic while a more hydrophobic material is located on selected portions of the opposing surface 360 (or vice versa, i.e., the material used to construct the opposing surface 360 may be hydrophobic and regions of that surface may be coated or otherwise treated to provide hydrophilic regions on the opposing surface).

Generally, the susceptibility of a solid surface to be wet out by a liquid is characterized by the contact angle that the liquid makes with the solid surface after being deposited on the horizontally disposed surface and allowed to stabilize thereon. It is sometimes referred to as the "static equilibrium contact angle," sometimes referred to herein merely as "contact angle". As discussed in U.S. Patent No. 6,372,954 B1 (Johnston et al.) and International Publication No. WO 99/09923 (Johnston et al.), the contact angle is the angle between a line tangent to the surface of a bead of liquid on a surface at its point of contact to the surface and the plane of the surface. A bead of liquid whose tangent was perpendicular to the plane of the surface would have a contact angle of 90 degrees.

For the purposes of the present invention, the hydrophilicity/hydrophobicity of surfaces are preferably determined on a relative scale such that if a component of the present invention is described as having hydrophobic and hydrophilic surfaces, the different surfaces are not necessarily either hydrophobic or hydrophilic. Both surfaces may, for example, be hydrophilic under conventional definitions, but one surface may be less hydrophilic than the other. Conversely, both surfaces may, for example, be hydrophobic under conventional definitions, but one surface may be less hydrophobic than the other. The "hydrophobic" and "hydrophilic" regions may, therefore, be described in terms of relative contact angle, e.g., the two surfaces may exhibit a difference in contact angle of 10 degrees or more (or, in some instances, 20 degrees or more) for drops of water at 20 degrees Celsius (even though both surfaces may conventionally be considered hydrophobic or hydrophilic). In other words, the hydrophobic surfaces of the present invention may exhibit a contact angle that is 10 degrees or more (or 20 degrees or more) higher than the contact angle of a hydrophilic surface (for water on a horizontal surface at 20 degrees Celsius).

As used herein, "hydrophilic" is used only to refer to the surface characteristics of a material, i.e., that it is wet by aqueous solutions, and does not express whether or not the material absorbs or adsorbs aqueous solutions. Accordingly, a material may be referred to as hydrophilic whether or not a layer of the material is impermeable or permeable to water or aqueous solutions.

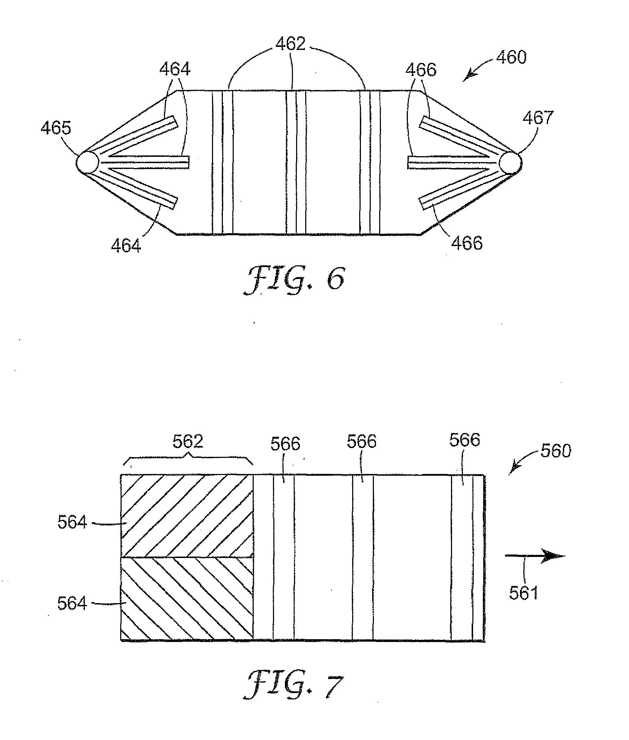

FIG. 6 is a plan view of another embodiment of an opposing surface 460 that may be used in a detection chamber of the present invention. The opposing surface 460 includes physical structures 462 in the form of channels that are preferably oriented generally perpendicular to the direction of flow through the detection chamber. In addition to the cross-chamber channels 462, the opposing surface 460 also includes flow directors 464 diverging outwardly towards the sides of the opposing surface 460 in a fan-shaped pattern at the inlet end 465. The opposing surface 460 depicted in Fig. 6 also includes flow directors 466 converging inwardly towards the center of the width of the width of the opposing surface 460 at the outlet end 467 of the opposing surface 460.

In use, the flow directors 464 at the inlet end 465 may preferably assist in expanding the flow front across the width of the opposing surface 460 (and, thus, the detection chamber in which the opposing surface 460 is located) as fluid enters the detection chamber. As the fluid reaches the first cross-chamber channel 462, the flow front may preferably stop moving in the direction of outlet end 467 until the flow front extends across the width the opposing surface 460. Once the flow front reaches across the opposing surface 460, it may preferably advance to the next cross-chamber channel 462 where it again halts until the flow front extends across the width of the opposing surface 460.

The flow front proceeds in the manner described in the preceding paragraph until reaching the optional flow directors 466 near the outlet end of the opposing surface 460. There the flow may preferably be directed to the outlet end 467 of the detection chamber where it can be directed to the waste chamber as described herein.

The flow control features depicted in FIG. 7 include an opposing surface 560 that includes an entry section 562 in which a series of channels 564 are oriented at an angle that is not perpendicular to the direction of fluid flow (as indicated by arrow 561). It may be preferred that the channels 564 diverge from a central axis 563 that generally bisects the width of the opposing surface 560 (where the width is measured generally perpendicular to the flow direction 561) and be arranged in a general V-shape with the width of the V-shape increasing along the flow direction and the vertex being located upstream from the opening. The channels 566 in second section of the opposing surface 560 may preferably be oriented generally perpendicular the fluid flow direction. Such an arrangement may be beneficial in ensuring fluid flow to the sides of the surface 560 and may also shunt or direct bubbles to the edges of the detection chamber where, e.g., they may not interfere with operation of the detection surface.

The variety of flow front control approaches described herein maybe used in combinations that are not explicitly described. For example, it may be preferred to use selected areas of hydrophobic and/or hydrophilic materials on the opposing surface in combination with physical structures (e.g., channels, discrete protruding structures, etc.) to provide control over the flow front progression through a detection chamber in the present invention. Further, although the interior volume of the detection chamber 530 may preferably have a generally rectilinear shape, it will be understood that detection chambers used in connection with the present invention may take other shapes, e.g., cylindrical, arcuate, etc.

Returning to FIG. 3, the optional staging chamber 220 that may also be included within the detection cartridge 210 may be used to stage, mix or otherwise hold sample material before its introduction to the detection chamber 230. The staging chamber 220 may take any suitable form. In some instances, it may be preferred that the volume of the staging chamber 220 be located above (relative to gravitational forces) the detection chamber 230 during use of the cartridge 210 such that static head can be developed within the sample material in the staging chamber 220 that can assist its passive delivery to the detection chamber 230 from the staging chamber 220.

An optional port 222 may be provided in the staging chamber 220 (or in another location that leads to the interior of the cartridge 210) such that material may be introduced into the interior volume of the cartridge 210 by, e.g., by syringe, pipette, etc. If provided, the port 222 may be sealed by, e.g., a septum, a valve, and/or other structure before and/or after materials are inserted into the cartridge 210. In some embodiments, the port 222 may preferably include, e.g., an external structure designed to mate with a test sample delivery device, e.g., a Luer lock fitting, threaded fitting, etc. Although only one port 222 is depicted, it should be understood that two or more separate ports may be provided.

In some embodiments, the staging chamber 220 may be isolated from direct fluid communication with the detection chamber 230 by a flow control structure/mechanism 224 (e.g., a valve). If a flow control structure/mechanism 224 is provided to isolate the detection chamber 230 from the staging chamber 220, then the staging chamber 220 may potentially be more effectively used to store materials before releasing them into the detection chamber 230. In the absence of a flow control structure/mechanism 224, some control over the flow of materials into the detection chamber 230 may potentially be obtained by other techniques, e.g., holding the cartridge 210 in an orientation in which the force of gravity, centripetal forces, etc. may help to retain materials in the staging chamber 220 until their delivery to the detection chamber 230 is desired.

Another optional feature depicted in FIG. 3 is the inclusion of a fluid monitor 227. The fluid monitor 227 may preferably provide for active, real-time monitoring of fluid presence, flow velocity, flow rate, etc, The fluid monitor 227 may take any suitable form, e.g., electrodes exposed to the fluid and monitored using e.g., alternating currents to determine flow characteristics and/or the presence of fluid on the monitors electrodes. Another alternative may involve a capacitance based fluid monitor that need not necessarily be in contact with the fluid being monitored.

Although depicted as monitoring the detection chamber 230, it should be understood that the fluid monitor may be located at any suitable location within the interior volume of the detection cartridge 210. For example, the fluid monitor could be located in the staging chamber 220, the waste chamber 240, etc. In addition, multiple fluid monitors may be employed at different locations within the cartridge 210.

Potential advantages of the fluid monitor 227 may include, e.g., the ability to automatically activate the introduction of sample materials, reagents, wash buffers, etc. in response to conditions sensed by the fluid monitor 227 that are employed in a feedback loop to, e.g., operate actuators 290 associated with modules 280, etc. Alternatively, the conditions sensed by the fluid monitor 227 can provide signals or feedback to a human operator for evaluation and/or action. For some applications, e.g., diagnostic healthcare applications, the fluid monitor 227 may be used to ensure that the detection cartridge is operating properly, i.e., receiving fluid within acceptable parameters.

Feedback loop control using the fluid monitor 227 may be accomplished using a controller outside of the cartridge 210 (see, e.g., the system of FIG. 11 or an embedded controller in the detection cartridge (see, e.g., FIGS. 1 & 2)). In use, the fluid monitor 227 may detect one or more conditions that could be used as the basis for delivering additional material to the interior of the detection cartridge 210 (into, e.g., staging chamber 220) using one or more modules 280 and/or input port 222.

Also depicted in FIG. 3 are optional modules 280 that may preferably be used to introduce or deliver materials into the cartridge 210 in addition to or in place of ports 222. It may be preferred, as depicted, that the modules 280 deliver materials into the staging chamber 220, although in some instances, they could potentially deliver materials directly into the detection chamber 230. The modules 280 may be used to deliver a wide variety of materials, although it may be preferred that the delivered materials include at least one liquid component to assist in movement of the materials from the module 280 and into the cartridge 210. Among the materials that could be introduced using modules 280 are, e.g., sample materials, reagents, buffers, wash materials, etc. Control over the introduction of materials from the modules 280 into the cartridge 210 may be obtained in a number of manners, e.g., the modules 280 may be isolated from the cartridge 210 by a seal, valve, etc. that can be opened to permit materials in the modules 280 to enter the cartridge 210.

It may be preferred that the modules 280 be independent of each other such that the materials contained within each module 280 can be introduced into the detection cartridge at selected times, at selected rates, in selected orders, etc. In some instances an actuator 290 may be associated with each module 280 to move the materials within the module 280 into the cartridge 210. The actuators 290 may be selected based on the design of the module 280. The actuators 290 may be manually operated or they may be automated using, e.g., hydraulics, pneumatics, solenoids, stepper motors, etc. Although depicted as a component of the detection cartridge 210, the actuators 290 may be provided as a part of the larger systems discussed herein (exemplary embodiments of which are depicted in FIGS. 1 & 2).

A potential advantage of using modules 280 to deliver materials such as reagents, buffers, etc, may be the opportunity to tailor the cartridge 210 for use with a wide variety of sample materials, tests, etc.

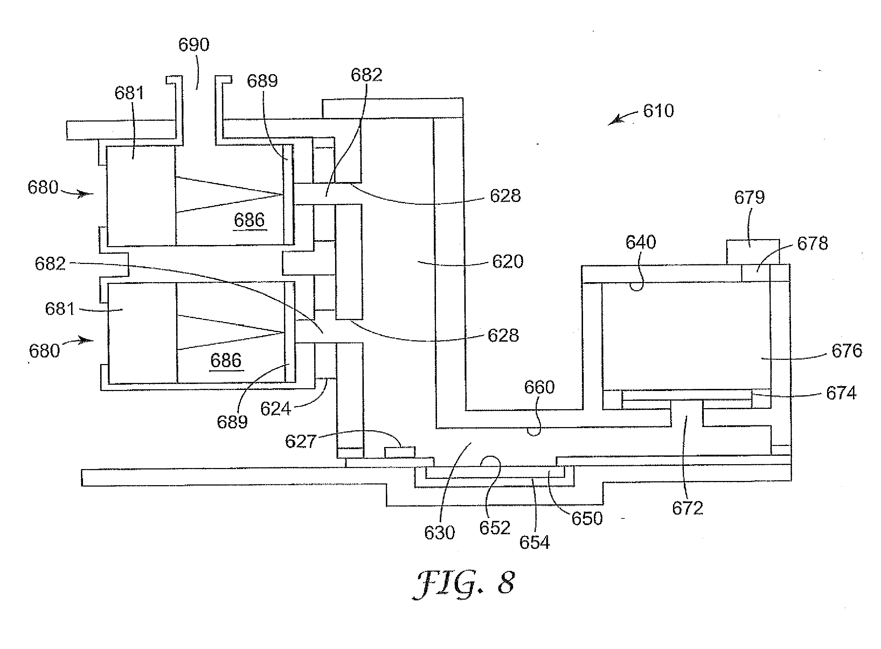

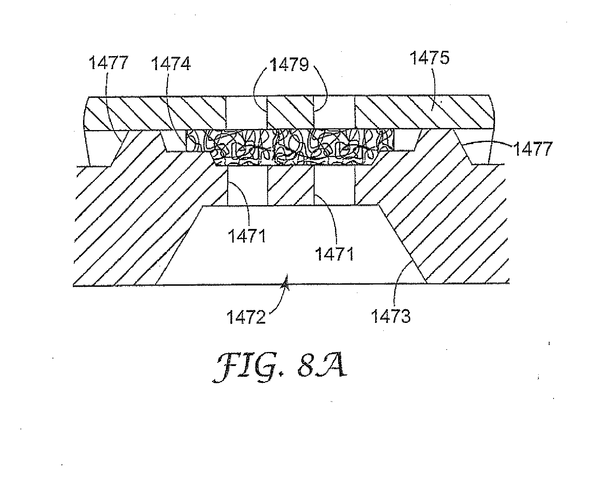

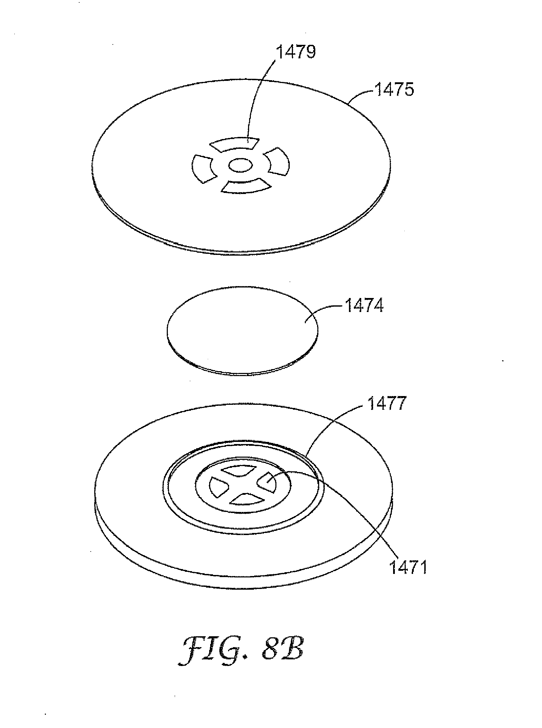

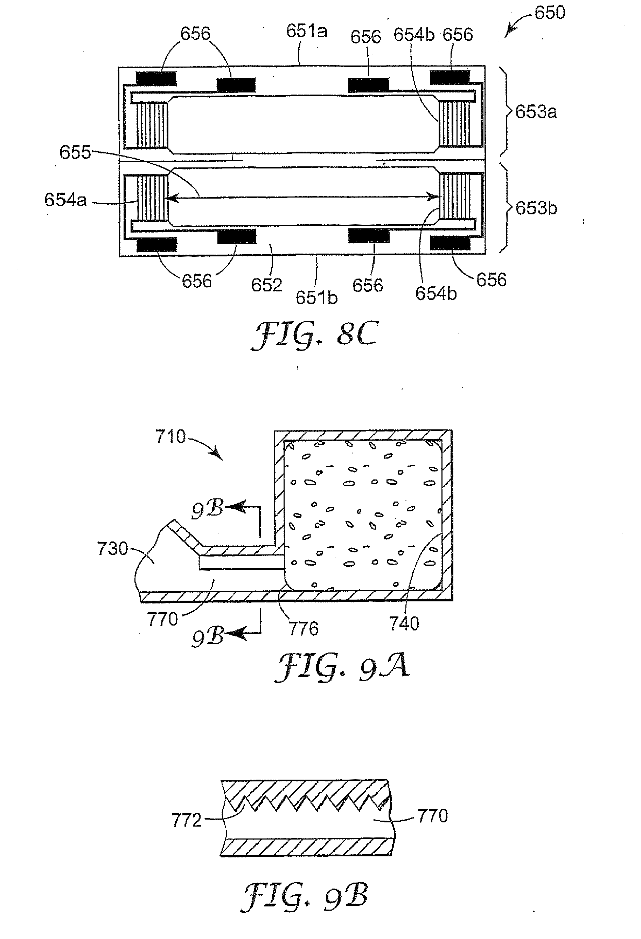

Various aspects of the detection cartridge 210 schematically depicted in FIG. 3 having been described, one exemplary embodiment of a detection cartridge 610 including a staging chamber 620, detection chamber 630 and waste chamber 640 is depicted in FIG. 8. The detection cartridge 610 includes a housing 612 and a sensor 650 having a detection surface 652 exposed within the detection chamber 630.

It may be preferred that the sensor 650 be an acousto-mechanical sensor such as, e.g., a Love wave shear horizontal surface acoustic wave sensor. As depicted, the sensor 650 may preferably be attached such that, with the possible exception of its perimeter, the backside 654 of the sensor 650 (i.e., the surface facing away from the detection chamber 630) does not contact any other structures within the cartridge 610.

Examples of some potentially suitable methods of attaching acousto-mechanical sensors within a cartridge that may be used in connection with the present invention may be found in, e.g., U.S. Patent Application Serial No. 60/533,176, filed on December 30,2003 as well as PCT Patent No. titled "Surface Acoustic Wave Sensor Asssznbiies", filed on even date herewith (Attorney Docket No. 58928US004).

In some instances, the processes used in the above-identified documents may be used with acoustic sensors that include contact pads that are exposed outside of the boundaries of a waveguide layer on the sensor using a Z-axis adhesive interposed between the sensor contact pads and traces on a carrier or support element to which the sensor is attached. Alternatively, however, the methods described in those documents may be used to make electrical connections through a waveguide layer where the properties (e.g., glass transition point (T_{g}) and melting point) of the Z-axis adhesive and the waveguide material are similar. In such a process, the waveguide material need not be removed from the contact pads on the sensor, with the conductive particles in the Z-axis adhesive making electrical contact through the waveguide material on the contact pads of the sensor.

It may be preferred that the portion of the detection surface 652 exposed within the detection chamber 630 be positioned to contact sample material flowing through the detection chamber 630. It may be preferred, for example, that the detection surface 652 be located at the bottom (relative to gravitational forces) of the detection chamber 630 such that materials flowing through the detection chamber 630 are urged in the direction of the detection surface 652 through at least the force of gravity (if not through other forces).

The detection chamber 630 may also preferably include an opposing surface 660 located opposite the detection surface 652. One or more different flow front control features may preferably be provided on the opposing surface 660 to assist in controlling the progression of a flow front through the detection chamber 630. Various examples of potentially suitable flow front control features are discussed herein.

It may be preferred that the opposing surface 660 and the detection surface 652 be spaced apart from each other such that the opposing surface 660 (and any protruding features located thereon) does not contact the detection surface 652. With respect to acoustic sensors, even close proximity may adversely affect the properties of the sensor operation if the opposing surface 660 disrupts the propagation of acoustic energy by the detection surface 652. It may be preferred, for example, that spacing between the detection surface 652 and the lowermost feature of the opposing surface 660 facing the active part of the detection surface 652 be 20 micrometers or more, or even more preferably 50 micrometers or more. For effective flow front control, it may be preferred that the distance between the lowermost feature of the opposing surface 660 and the detection surface 652 be 10 millimeters, alternatively 1 millimeter or less, in some instances 500 micrometers or less, and in other instances 250 micrometers or less.

The cartridge 610 of FIG. 8 also includes a waste chamber 640 that is in fluid communication with the detection chamber 630 and into which sample material flows after leaving the detection chamber 630. The cartridge 610 may preferably include a volumetric flow control feature interposed in the fluid path between the detection chamber 630 and the waste chamber 640. The volumetric flow control feature may preferably function to control the rate at which sample material from the detection chamber 630 flows into the waste chamber 640.

Although the volumetric flow control feature may take many different forms, in the embodiment depicted in FIG. 6 it is provided in the form of an opening 672 over which a capillary structure in the form of a porous membrane 674 is located. In addition to the porous membrane 674, a mass of absorbent material 676 is located within the waste chamber 640.

The porous membrane 674 may preferably provide a fluid pressure drop from the side facing the detection chamber 630 to the side facing the waste chamber 640. The porous membrane 674 preferably assists in controlling the flow rate from the detection chamber 630 into the waste chamber 640. The pressure drop may preferably be provided by capillary action of the passageways within the porous membrane 674. The pressure drop across a porous membrane is typically a function of the pore size and the thickness of the membrane. It may be preferred that the porous membrane have a pore size in the range of, e.g., 0.2 microns to 50 microns. Some suitable examples of materials that may be useful as a porous membrane include, e.g., acrylic copolymers, nitrocellulose, polyvinylidene fluoride (PVDF), polysulfone, polyethersulfone, nylon, polycarbonate, polyester, etc.

Referring to FIGS. 8A & 8B, an alternative structure using a porous membrane 1474 to control fluid flow rate into a waste chamber is depicted. The opening 1472 includes a series of orifices 1471 formed through the material of the housing. The opening 1472 may preferably include a chamfer 1473 to preferably assist in fluid flow through the opening 1472 by avoiding a sharp edge that may inhibit flow into and through the opening 1472 (alternatively, radiused, rounded or smoothed edges, etc. could be used).

The porous membrane 1474 is held in place by a cover plate 1475 that, in the preferred embodiment may be ultrasonically welded over the orifices 1471 with the porous membrane 1474 located therebetween. The cover plate 1475 may preferably include orifices 1479 through which fluids may pass into a waste chamber. The ultrasonic welding of the cover plate 1475 maybe assisted by the use of an energy director 1477 surrounding the opening 1472 and the height of the energy director 1477 may be sufficient to allow some clearance for the thickness of the porous membrane 1474. In such a system, the cover plate 1475 and energy director 1477 may assist in the formation of a fluid-tight attachment without destruction of the porous membrane 1474. Other techniques for retaining the membrane 1474 over opening 1472 may also be used, e.g., adhesives, thermal welding, solvent welding, mechanical clamping, etc. These techniques may be used with or without a cover plate 1475, i.e., the porous membrane 1474 itself may be directly attached to the structures surrounding the opening 1472.

Referring again to FIG. 8, although the membrane 674 may draw fluid from the detection chamber 630, surface tension in the fluid may prevent the fluid from flowing out of the membrane 674 and into the waste chamber 640. As a result, it may be preferred to draw fluid from the membrane 674 into the waste chamber 640 using, e.g., negative fluid pressure within the waste chamber 640. The negative fluids pressure within the waste chamber 640 may be provided using a variety of techniques. One technique for providing a negative fluid pressure within the waste chamber 640 may include, e.g., absorbent material 676 located within the waste chamber 640 as depicted in FIG. 8. One alternative technique for providing a negative fluid pressure within the waste chamber 640 is a vacuum within the waste chamber 640. Other alternative techniques may also be used.

It may be preferred that negative fluid pressure within the waste chamber 640 be provided passively, e.g., through the use of absorbent material or other techniques that do not require the input of energy (as would, for example, maintaining a vacuum within the waste chamber). Examples of some potentially suitable absorbent materials that may provided within the waste chamber 640 may include, but are not limited to, foams (e.g., polyurethane, etc.), particulate materials

(e.g., alumina-silicate, polyacrylic acid, etc.), granular materials (e.g., cellulose, wood pulp, etc.).

If the waste chamber 640 is provided with absorbent material 676 located therein as depicted in FIG. 8, it may be preferred that the absorbent material be in physical contact with the side of the membrane 674 (or any orifices 1479 in a cover plate 1475 as seen in FIGS. 8A & 8B) facing the interior of the waste chamber 640. A gap between the absorbent material 676 and the membrane 674 may limit or prevent fluids from leaving the membrane 674 and entering the waste chamber 640 because of, e.g., surface tension within the fluid as contained in the membrane 674.

If the waste chamber 640 is provided with absorbent material 676 located therein as depicted in FIG. 8, it may be preferred that the absorbent material be in physical contact with the side of the membrane 674 facing the interior of the waste chamber 640. A gap between the absorbent material 676 and the membrane 674 may limit or prevent fluids from leaving the membrane 674 and entering the waste chamber 640 because of, e.g., surface tension within the fluid as contained in the membrane 674.

If absorbent material 676 is provided within the waste chamber 640, it may be beneficial to provide a variety of layers of absorbent materials to control the volumetric flow rate into the waste chamber 640. For example, a first layer of absorbent material may be provided proximate the membrane 674, with the first layer material having a characteristic wicking rate and a defined fluid volume. After the first layer of absorbent material has been loaded to its capacity, the fluid entering the waste chamber 640 may be drawn into a second layer of absorbent material with a different wicking rate, thereby potentially providing a different negative pressure in the waste chamber 640.

Changing the negative pressure within the waste chamber 640 using, e.g., different layers of absorbent materials, may be used to compensate for other changes within the cartridge 610 such as, e.g., changes in fluid head pressure as sample material is drawn through the cartridge 610. Other techniques may also be used to compensate for changes in the fluid head pressure such as, e.g., changing a vacuum level held in the waste chamber, opening one or more vents in the cartridge, etc.

The embodiment of FIG. 8 includes a vent 678 in the waste chamber 640 that may place the interior volume of the waste chamber 640 in communication with ambient atmosphere. Opening and/or closing the vent 678 may be used to control fluid flow into the waste chamber 640 and, thus, through the cartridge 610. Furthermore, the vent 678 may be used to reduce pressure within the waste chamber 640 by, e.g., drawing a vacuum, etc. through the vent 678,

Although depicted as being in direct fluid communication with the waste chamber 640, one or more vents may be provided and they may be directly connected to any suitable location that leads to the interior volume of the detection cartridge 610, e.g., staging chamber 620, detection chamber 630, etc. The vent 678 may take any suitable form, e.g., one or more voids, tubes, fitting, etc.

The vent 678 may include a closure element 679 in the form of a seal, cap, valve, or other structure(s) to open, close or adjust the size of the vent opening, If provided as a seal, the seal may be adhesively or otherwise attached over or located within the vent 678. In some embodiments, the closure element 679 may be used to either open or close the vent. In other embodiments, the closure element 679 may be adjustable such that the size of the vent opening may be adjusted to at least one size between fully closed and fully open to adjust fluid flow rate through the detection cartridge 610. For example, increasing the size of the vent opening may increase fluid flow rate while restricting the size of the vent opening may cause a controllable reduction the fluid flow rate through the interior volume of the detection cartridge 610, e.g., through the staging chamber 620, detection chamber 630, etc. For example, the vent 678 may be provided with a flow restrictor that can be used to adjust the vent opening size. If the vent 678 includes multiple orifices, one or more of the orifices can be opened or closed to control fluid flow, etc.

FIG. 8C is a view of the detection surface 652 of one potential sensor 650 that may be used in connection with the present invention. Although depicted in connection with a detection cartridge, it should be understood that the sensor design depicted in FIG. 8C could be used in any acousto-mechanical sensor. The detection surface 652 includes two channels 653a and 653b, each of which includes a pair of interdigitated transducers 654a and 654b (respectively) similar to known transducers used to excite piezoelectric substrates in acousto-mechanical sensors.

The channels 653a and 653b are, however, different from known sensors in that the acoustic pathlength 655 as measured between the opposing transducers 654a and 654b is enhanced because the contact pads 656 used to deliver electrical energy to the transducers 654a and 654b are located off to one side of the acoustic path defined between the transducers 654a and 654b in each of the channels 653a and 653b. Because the contact pads 656 are located off to one side of the acoustic path, the contact pads 656 can be located between the ends of the acoustic path (as defined by the transducers 654a and 654b at each end of each channel 653a and 653b). The contact pads 656 are connected to the electrodes 654a and 654b by leads as depicted in FIG. 8C.

Locating the contact pads 656 off to one side of the acoustic path of each channel 653a and 653b may be beneficial because the acoustic pathlength can be increased by moving the transducers 654a and 654b farther apart on a given detection surface 652. Where two channels 653a and 653b are to be formed on the same detection surface 652, it may be preferred that the contact pads 656 are not located between two acoustic paths of the channels 653a and 653b, but rather off to the sides of the two acoustic paths (e.g., a primary acoustic path and a secondary acoustic path) as depicted in FIG. 8C.

Although each acoustic path on the substrate of FIG. 8C is defined by a pair of transducers as would be typical for, e.g., a delay line sensor, it should be understood that the principles depicted in FIG. 8C could be implemented as well in a sensor that includes only one transducer arranged to operate as a resonator device. In such a device, the contact pads connected to the transducer would preferably be off to one side of and between the ends of the acoustic path defined by the one transducer.

FIGS. 9A & 9B depict a portion of an alternative cartridge 710 including a portion of a detection chamber 730 and a waste chamber 740. The waste chamber 740 and the detection chamber 730 are, in the depicted embodiment, separated by a capillary structure in the form of a flow passage 770 that includes a set of capillary channels 772 that may preferably draw fluid from the detection chamber 730 by capillary forces. The particular shape of the capillary channels 772 may be different from those depicted in the cross-sectional view of FIG. 9B. Also, the number of capillary channels 772 provided in the flow passage may vary from as few as one capillary channel to any selected number of multiple capillary channels.

In the embodiment of FIGS. 9A & 9B, the flow passage 770 may preferably take the place of the porous membrane used in connection with the embodiment of FIG. 8. The capillary channel or channels 770 preferably provide the desired level of negative fluid pressure to draw fluid from the detection chamber 730.

In some instances, it may be preferred to provide both a porous membrane and one or more capillary channels to provide a capillary structure between the detection chamber and the waste chamber in detection cartridges of the present invention. Other capillary structures such as tubes, etc. could be substituted for the exemplary embodiments described herein.

Although the capillary channels 772 may draw fluid from the detection chamber 730, surface tension in the fluid may prevent the fluid from flowing out of the flow passage 770 and into the waste chamber 740. As a result, it may be preferred to draw fluid from the flow passage 770 into the waste chamber 740 using, e.g., negative fluid pressure within the waste chamber 740. The negative fluid pressure within the waste chamber 740 may be provided using a variety of techniques. One technique for providing a negative fluid pressure within the waste chamber 740 may include, e.g., absorbent material 776 located within the waste chamber 740 as depicted in FIG. 9A. One alternative technique for providing a negative fluid pressure within the waste chamber 740 is a vacuum within the waste chamber 740. Other alternative techniques may also be used.

It may be preferred that negative fluid pressure within the waste chamber 740 be provided passively, e.g., through the use of absorbent material or other techniques that do not require the input of energy (as would, for example, maintaining a vacuum within the waste chamber). The use of absorbent materials within a waste chamber is described above in connection with the embodiment depicted in FIG. 8.

If absorbent materials are used within the waste chamber 740, it may be preferred that the absorbent material be in contact with the end or ends of any capillary channel(s) 772 to overcome any surface tension that might otherwise prevent fluid from exiting the capillary channel(s).

Referring again to the cartridge depicted in FIG. 8, the staging chamber 620 may be provided upstream from the detection chamber 630. The staging chamber 620 may provide a volume into which various components may be introduced before entering the detection chamber 630. Although not depicted, it should be understood that the staging chamber 620 could include a variety of features such as, e.g., one or more reagents located therein (e.g., dried down or otherwise contained for selective release at an appropriate time); coatings (e.g., hydrophilic, hydrophobic, etc.); structures/shapes (that may, e.g., reduce/prevent bubble formation, improve/cause mixing, etc.).

Also, the fluid path between the staging chamber 620 and the detection chamber 630 may be open as depicted in FIG. 8. Alternatively, the fluid path between the staging chamber 620 and the detection chamber 630 may include a variety features that may perform one or more functions such as, e.g., filtration (using, e.g., porous membranes, size exclusion structures, beads, etc.), flow control (using, e.g., one or more valves, porous membranes, capillary tubes or channels, flow restrictors, etc.), coatings (e.g., hydrophilic, hydrophobic, etc.), structures/shapes (that may, e.g., reduce/prevent bubble formation and/or transfer, improve mixing, etc.).

Another optional feature depicted in FIG. 8 is the inclusion of a fluid monitor 627 in the flow path between the staging chamber 620 and the detection chamber 630. The fluid monitor 627 may preferably provide for active, real-time monitoring of fluid presence, flow velocity, flow rate, etc. The fluid monitor 627 may take any suitable form, e.g., electrodes exposed to the fluid and monitored using e.g., alternating currents to determine flow characteristics and/or the presence of fluid on the monitors electrodes. Another alternative may involve a capacitance based fluid monitor that need not necessarily be in contact with the fluid being monitored.

Potential advantages of the fluid monitor 627 may include, e.g., the ability to automatically activate the introduction of sample materials, reagents, wash buffers, etc. in response to conditions sensed by the fluid monitor 627. Alternatively, the conditions sensed by the fluid monitor 627 can provide signals or feedback to a human operator for evaluation and/or action. For some applications, e.g., diagnostic healthcare applications, the fluid monitor 627 may be used to ensure that the detection cartridge is operating properly, i.e., receiving fluid within acceptable parameters.

Feedback loop control using the fluid monitor 627 may be accomplished using a controller outside of the cartridge 610 (see, e.g., the system of FIG. 11 or an embedded controller in the detection cartridge (see, e.g., FIGS. 1 & 2)). In use, the fluid monitor 627 may detect one or more conditions that could be used as the basis for delivering additional material to the interior of the detection cartridge 610 (into, e.g., staging chamber 620) using one or more modules 680.

The exemplary cartridge 610 depicted in FIG. 8 includes two modules 680 arranged to deliver material into the staging chamber 620 of the cartridge 610. The modules 680 deliver their materials into the staging chamber 620 through module ports 628 that open into the staging chamber 620 (it should be understood that the orientation or direction of the modules 680 with respect to the staging chamber 620 may vary from that depicted). The modules 680 may preferably be attached to the module ports 628 by an adhesive 624 or other material capable of providing a suitable fluid-tight seal between the modules 680 and the module ports 628. Any suitable technique for attaching the modules 680 to the module ports 628 may be substituted for the adhesive 624. In some instances, the modules 680 may be welded (chemically, thermally, ultrasonically, etc.) or otherwise attached over the module ports 628. In other instances, the modules 680 may be connected to the module ports using complementary structures such as threaded fittings, Luer locks, etc.

Although other exemplary embodiments of modules that may be used to introduce materials into the cartridge 610 are described elsewhere, each of the modules 680 depicted in FIG. 8 includes a seal 689 over an opening 682 that is aligned over the module port 628 leading into staging chamber 620. Each of the modules 680 also includes a plunger 681 that defines a chamber 686 located between the seal 689 and the plunger 681. The material or materials to be delivered into the staging chamber 620 are typically located within the chamber 686 before the plunger 681 is used to deliver the contents of the module 680 into the staging chamber 620.

In the depicted embodiment, the plunger 681 may preferably be designed to pierce, tear or otherwise open the seal 689 to allow the materials with the module 680 to enter the staging chamber 620. The depicted plungers 681 include piercing tips for that purpose. It should be understood that the modules 680 could alternatively be isolated from the staging chamber 620 by valves or any other suitable fluid structure used to control movement of materials between chambers.

One variation depicted in FIG. 8 is that the upper module 680 includes a port 690 opening into the chamber 686 of the module 680. The port 690 may be used to deliver materials into the chamber 686 for subsequent delivery to the staging chamber 620 using the module 680. For example, the port 690 may be used to introduce a collected specimen, etc. into the module 680 where it can then be introduced into the staging chamber 620 at selected times and/or rates. In addition, the chamber 686 of the module 680 receiving the sample material may include one or more reagents or other materials that contact the sample material upon its introduction to the module 680. Although not depicted, it may be preferred that the port 690 be sealed before and/or after sample material is introduced into the module 680. The port 690 may be sealed by, e.g., a septum, a valve, induction welded seal, cap, and/or other structure before and/or after materials are inserted into the module 680.

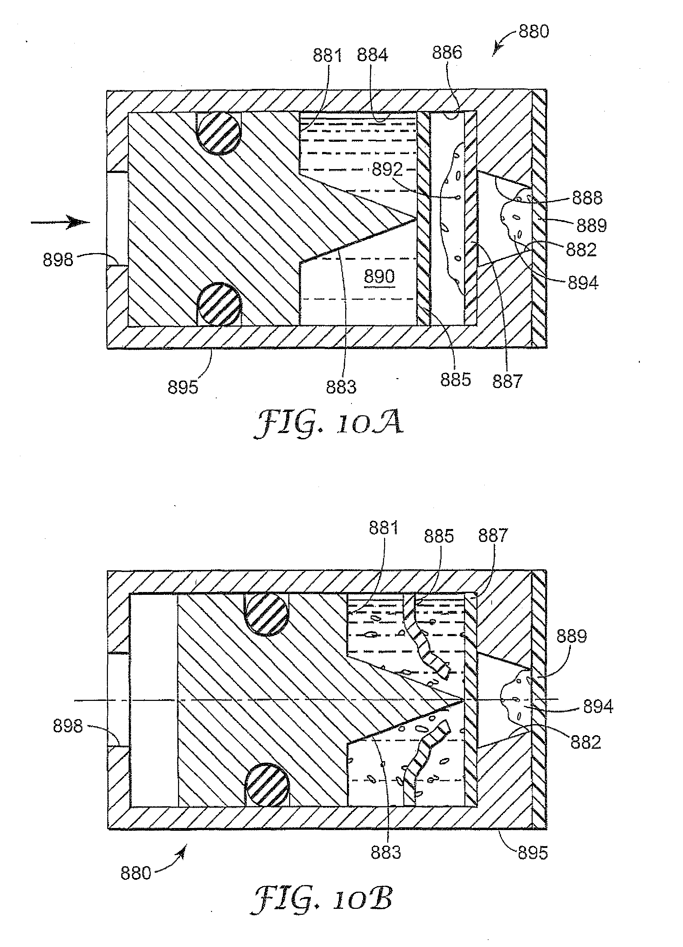

One exemplary embodiment of a module 880 that may be used to deliver reagents and/or other materials in accordance with the present invention is depicted in the cross-sectional views of FIGS. 10A & 10B. The depicted exemplary module 880 includes multiple chambers, each of which may contain the same or different materials and each of which may preferably be hermetically sealed from each other. It may be preferred that the module 880 be designed such that the materials within the different chambers mix as they are introduced to each other.

By storing the different materials within separate chambers, it may be possible to provide materials in the module 880 that are preferably not mixed until needed. For example, some substances may preferably be stored in a dry state to, e.g., prolong their shelf life, usable life, etc., but the same substances may need to be mixed in liquids that may include water, etc. to provide a usable product. By providing the ability to mix and/or dispense these materials on demand, the modules of the present invention can provide a convenient storage and introduction device for many different materials.

The depicted module 880 includes three chambers 884, 886 and 888. The chambers may preferably be separated by a seal 885 (located between chambers 884 and 886) and seal 887 (located between chambers 886 and 888). The depicted module 880 also includes plunger 881 with a tip 883 that, in the depicted embodiment, is designed to pierce seals 885 and 887 as the plunger 881 is moved from the loaded position depicted in FIG. 10A (i.e., on the left end of the module 880) to the unloaded position (i.e., towards the exit port 882 as indicated by the arrow in FIG. 10A). The plunger 881 may preferably include an o-ring (depicted) or other sealing structure to prevent materials in the chambers from moving past the plunger 881 in the opposite direction, i.e., away from the opening 882.

FIG. 10B depicts a dispensing operation in which the plunger 881 is in transit from the loaded position of FIG. 10A to the unloaded position. In FIG. 10B, the tip 883 has pierced seal 885 such that the materials in chambers 884 and 886 can contact each other and mix. It may be preferred that chamber 884 contain a liquid 890, e.g., water, saline, etc. and that chamber 686 contain a dried-down reagent 692 (e.g., a lysing agent, fibrinogen, etc.), with the liquid 890 causing the reagent 892 to enter into a solution, suspension, mixture, etc. with the liquid 890. Although reagent 892 is depicted as being dried-down within chamber 886, it may be located in, e.g., a powder, gel, solution, suspension, or any other form. Regardless of the form of the materials in the chambers 884 and 886, piercing or opening of the seal 885 allows the two materials to contact each other and preferably mobilize within module 880 such that at least a portion can be delivered out of the module 880.

As the plunger 881 is advanced towards the exit port 882, the tip 883 also preferably pierces seal 887 such that the materials 894 in the chamber 888 can preferably contact the materials 890 and 692 from chambers 884 and 886.

When fully advanced towards the exit port 882, the tip 883 may preferably pierce exit seal 889 provided over exit port 882, thereby releasing the materials 890, 892 and 894 from fluid module 880 and into, e.g., a staging chamber or other space. It may be preferred that the shape of the plunger 881 and tip 883 mate with the shape of the final chamber 888 and exit port 882 such that substantially all of the materials in the various chambers are forced out of the fluid module 880 when the plunger 881 is advanced completely through the fluid module 880 (i.e., all of the way to the right of FIGS. 10A & 10B).

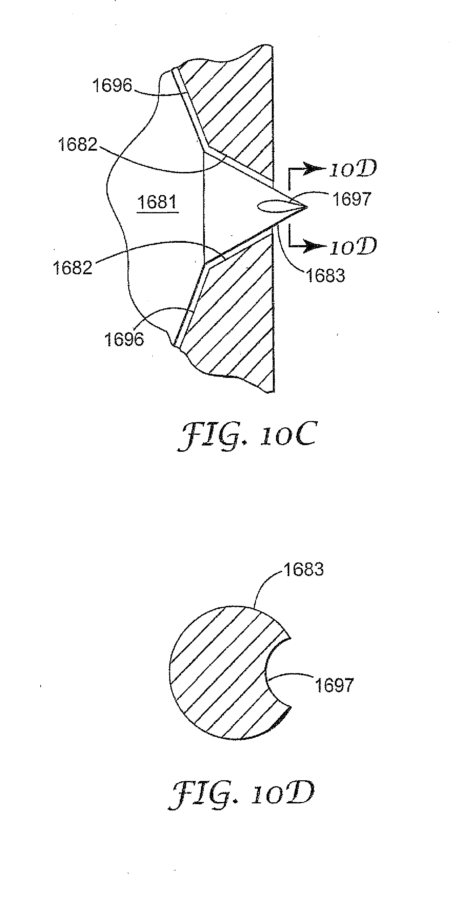

FIG. 10C is an enlarged view of on exemplary alternative tip 1683 in the opening 1682 of a module. The tip 1683 preferably extends from a plunger 1681. As discussed herein, the shape of the tip 1683 and plunger 1681 may preferably mate with the shape of the opening 1682 in the module housing 1695. For example, the portion of the depicted tip 1683 has a conical shape that confirms to the frusto-conical shape of the opening 1682, In addition, it may be preferred that the plunger 1681 and the inner surface 1696 of the module facing the plunger 1681 also conform to each other. Conformance between the plunger 1681 and tip 1683 with the mating features of the module may enhance complete delivery of materials from the module into the cartridges of the present invention.

Furthermore, it may be preferred that the tip 1683 be provided in a shape or with features that facilitate the transfer of materials past the seals pierced by the tip 1683. The feature may be as simple as a channel 1697 formed in an otherwise conical tip 1683 as depicted in FIGS. 10C & 10D. Alternatively, the tip 1683 itself may have many other shapes to reduce the likelihood that the tip will form a barrier to fluid flow with a seal it pierces. Such alternatives may include, e.g., star-shaped piercing tips, ridges, etc.

The plunger 881 in module 880 may be moved by any suitable actuator or technique. For example, the plunger 881 may he driven by a mechanical device (e.g., piston) inserted into module 880 through driver opening 898 or fluid pressure may be introduced into module 880 through driver opening 898 to move the plunger 881 in the desired direction. It may be preferred to drive the plunger 881 using, e.g., a stepper motor or other controlled mechanical structure to allow for enhanced control over the movement of plunger 881 (and any associated structure such as, e.g., tip 883). Other means for moving plunger 881 will be known to those skilled in the art, e.g., solenoid assemblies, hydraulic assemblies, pneumatic assemblies, etc.

The module 880, plunger 881 and tip 883 may be constructed of any suitable material or materials, e.g., polymers, metals, glasses, silicon, ceramics, etc. that provide the desired qualities or mechanical properties and that are compatible with the materials to be stored in the modules. Similarly, the seals 885, 887 and 889 may be manufactured of any suitable material or materials, e.g., polymers, metals, glasses, etc. For example, the seals may preferably be manufactured from polymer film/metallic foil composites to provide desired barrier properties and compatibility with the various materials to be stored in the module 880.

It may be preferred that the materials used for both the seals and the module housing be compatible with the attachment technique or techniques used to attach the seals in a manner that prevents leakage between the different chambers. Examples of some attachment techniques that that may be used in connection with modules 880 include, e.g., heat sealing, adhesives, chemical welding, heat welding, ultrasonic welding, combinations thereof, etc. It should also be understood that the modules may be constructed such that the seals are held in place by friction, compression, etc. Furthermore, it should be understood that in some embodiments, it may be possible to open the seals in a fluid module without the use of tip or other structure that pierces the seals. For example, the seals may be opened through fluid pressure alone (i.e., the seals may be designed to burst under pressure as the plunger is moved from the loaded position towards the exit port using, e.g., a line of weakness formed in the seal, etc.).

### SYSTEM DESIGN

It may desirable that the detection cartridges of the present invention be capable of docking with or being connected to a unit that may, e.g., provide a variety of functions such as providing power to the sensors or other devices in the detection cartridge, accepting data generated by the sensor, providing the ability to take user input to control fluid flow and/or sensor operation, etc.

One such system 900 is schematically depicted in FIG. 11, and may preferably include a power source 901 and user interface 902 (e.g., pushbuttons, keyboard, touchscreen, microphone, etc.). The system 900 may also include an identification module 903 adapted to identify a particular detection cartridge 910 using, e.g., barcodes, radio-frequency identification devices, mechanical structures, etc.

The system 900 may also preferably include a sensor analyzer 904 that obtains data from a sensor in the detection cartridge and a processor 905 to interpret the output of the sensor. In other words, sensor analyzer 904 may receive output from a sensor detection cartridge 910 and provide input to processor 905 so that the output of the sensor can be interpreted.

Processor 905 receives input from sensor analyzer 904, which may include, e.g., measurements associated with wave propagation through or over an acousto-mechanical sensor. Processor 905 may then determine whether a target biological analyte is present in sample material. Although the invention is not limited in this respect, the sensor in detection cartridge 910 may be electrically coupled to sensor analyzer 904 via insertion of the detection cartridge 910 into a slot or other docking structure in or on system 900. Processor 905 may be housed in the same unit as sensor analyzer 904 or may be part of a separate unit or separate computer.

Processor 905 may also be coupled to memory 906, which can store one or more different data analysis techniques. Alternatively, any desired data analysis techniques may be designed as, e.g., hardware, within processor 905. In any case, processor 905 executes the data analysis technique to determine whether a detectable amount of a target biological analyte is present on the detection surface of a sensor in detection cartridge 910.

By way of example, processor 905 maybe a general-purpose microprocessor that executes software stored in memory 906. In that case, processor 905 may be housed in a specifically designed computer, a general purpose personal computer, workstation, handheld computer, laptop computer, or the like. Alternatively, processor 905 maybe an application specific integrated circuit (ASIC) or other specifically designed processor. In any case, processor 905 preferably executes any desired data analysis technique or techniques to determine whether a target biological analyte is present within a test sample.

Memory 906 is one example of a computer readable medium that stores processor executable software instructions that can be applied by processor 905. By way of example, memory 906 may be random access memory (RAM), read-only memory (ROM), non-volatile random access memory (NVRAM), electrically erasable programmable read-only memory (EEPROM), flash memory, or the like. Any data analysis techniques may form part of a larger software program used for analysis of the output of a sensor (e.g., LABVIEW software from National Instruments Corporation, Austin, Texas).

Further descriptions of systems and data analysis techniques that may be used in connection with the present invention (to provide, e.g., means for driving sensors and/or means for analyzing data from the sensors) may be described in, e.g., U.S. Patent Application Serial No. 60/533,177, filed on December 30,2003, and PCT Patent No. _, titled "Estimating Propagation Velocity Through A Surface Acoustic Wave Sensor", filed on even date herewith (Attorney Docket No. 58927WO003), Other data analysis techniques to determine the presence (or absence) of target biological analytes using sensors of the invention may also be sued, e.g., time domain gating used as a post-experiment noise reduction filter to simplify phase shift calculations, etc. Still other potentially useful data analysis techniques may be described in the documents identified herein relating to the use of acoustic sensors. Although systems and methods related to the use of surface acoustic wave sensors are described therein, it should be understood that the use of these systems and methods may be used with other acousto-mechanical sensors as well.

### MANUFACTURING ACOUSTO-MECHANICAL SENSORS

As discussed herein, the present invention relies on the use of acousto-mechanical sensors to detect the presence of target biological analyte within a test sample flowed over a detection surface. Coating or otherwise providing the various materials needed to provide acousto-mechanical sensors with the desired selective attachment properties may be performed using a variety of methods and techniques.

One example of a potentially useful construction is depicted in FIG. 12 and includes a substrate 1080 on which a waveguide 1082 is located. A tie layer 1084 may be provided between an immobilization chemistry layer 1086 and waveguide 1082 if necessary to, e.g., obtain the desired level of adhesion between those layers (or to achieve some other result). A layer of capture agents 1088 may be provided on the immobilization layer 1086 and, in some embodiments, a passivation layer 1090 may be provided over the layer of capture agents 1088.

As used with acoustic sensors, the waveguide materials, immobilization materials, capture agents, etc, used on the sensors may be deposited by any suitable technique or method. Typically, it may be preferred that such materials be delivered to a substrate in a carrier liquid, with the carrier liquid and the materials forming, e.g., a solution or dispersion. When so delivered, examples of some suitable deposition techniques for depositing the materials on a surface may include, but are not limited to, flood coating, spin coating, printing, non-contact depositing (e.g., ink jetting, spray jetting, etc.), pattern coating, knife coating, etc. It may be preferred, in some embodiments, that the deposition technique have the capability of pattern coating a surface, i.e., depositing the materials on only selected portions of a surface. U.S. Patent Application Serial No. 10/607,698, filed June 27, 2003, describes methods of pattern coating that may be suitable for use in connection with the construction of sensors according to the present invention.

In some embodiments, (such as those described in, e.g., PCT Patent No. _, titled "Acoustic Sensors and Methods", filed on even date herewith (Attorney Docket No. 60209WO003) and others), some materials may function as both waveguide material and immobilization material for secondary capture agents on an underlying substrate. In other embodiments, the same materials may function as waveguide material, immobilization material, and capturing materials. In both of these variations, the materials of the present invention may preferably be deposited on an underlying substrate that is, itself, effectively insoluble in the carrier liquid such that the carrier liquid does not adversely affect the underlying substrate.

If, however, the surface on which the waveguide materials, immobilization materials, and/or capture agents are to be deposited exhibits some solubility in the carrier liquid used to deliver the material, it may be preferred that the material be deposited using a non-contact deposition technique such as, e.g., ink jetting, spray jetting etc. For example, if the underlying substrate is a waveguide formed of, e.g., polyimide, acrylate, etc., on a sensor substrate and the material of an immobilization layer is to be deposited using, e.g., butyl acetate, as the carrier liquid, then it may be preferred to use a non-contact deposition method to limit deformation of the waveguide and to preferably retain the functional characteristics of the immobilization material exposed on the resulting coated surface. The same considerations may apply to the coating of capture agents on a surface.

There are several variables that may be controlled in a spray-jet coating process, including deposition rate, substrate speed (relative to the spray jet head), sheath gas flow rate, sheath gas, raster spacing, raster pattern, number of passes, percent solids in the sprayed solution/dispersion, nozzle diameter, the carrier liquid, the composition of the underlying surface on which the materials of the present invention are being deposited, etc. Specific conditions under which the materials of the present invention can be deposited to yield a suitable coating may be determined empirically.

As used herein and in the appended claims, the singular forms "a," "and," and "the" include plural referents unless the context clearly dictates otherwise. Thus, for example, reference to "a target biological analyte" includes a plurality of target biological analytes and reference to "the detection chamber" includes reference to one or more detection chambers and equivalents thereof known to those skilled in the art.

The terms "comprises" and variations thereof do not have a limiting meaning where these terms appear in the description or the claims.

All references and publications identified herein are expressly incorporated herein by reference in their entirety into this disclosure. Illustrative embodiments of this invention are discussed and reference has been made to possible variations within the scope of this invention. These and other variations and modifications in the invention will be apparent to those skilled in the art without departing from the scope of the invention, and it should be understood that this invention is not limited to the illustrative embodiments set forth herein. Accordingly, the invention is to be limited only by the claims provided below and equivalents thereof

## Claims

1. A shear horizontal surface acoustic wave sensor comprising:
a piezoelectric substrate comprising a major surface;
at least one transducer on the major surface of the piezoelectric substrate, wherein the at least one transducer defines an acoustic path on the major surface of the piezoelectric substrate, wherein the acoustic path comprises a first end and a second end;
wherein the at least one transducer comprises a contact pad on the major surface of the piezoelectric substrate, wherein the contact pad is located off to a first side of the acoustic path and between the first end and the second end of the acoustic path, wherein the contact pad is connected to the at least one transducer by a lead.

2. A sensor according to claim 1, wherein the at least one transducer comprises a pair of contact pads on the major surface of the piezoelectric substrate, wherein the pair of contact pads are located off to the first side of the acoustic path and between the first end and the second end of the acoustic path, wherein the contact pads are each connected to the at least one transducer by a lead.
